# EUROPEAN PATENT SPECIFICATION

(11) **EP 4 294 342 B1**
(45) Date of publication and mention of the grant of the patent: **26.02.2025**
(21) Application number: 22701429.7
(22) Date of filing: 20.01.2022
(51) Int. Cl.: A61F 13/05, A61M 1/00

(54) **METHODS OF FORMING A MANIFOLD PAD**
VERFAHREN ZUR HERSTELLUNG EINES LEITUNGSSYSTEMSKISSENS
MÉTHODES DE FORMATION D'UN COUSSINET DISTRIBUTEUR

(30) Priority: 18.02.2021 US 202163150996 P
(43) Date of publication of application: 27.12.2023
(73) Proprietor: KCI Manufacturing Unlimited Company, Athlone, Co. Westmeath, N37XF22 (IE)
(72) Inventor: REHBEIN, Jonathan, San Antonio, Texas 78265 (US); GONZALEZ, Javier, San Antonio, Texas 78265 (US)
(74) Representative: Simmons & Simmons
(86) International application number: PCT/IB2022/050471
(87) International publication number: WO 2022/175765

(56) References cited:
- WO-A1-2019/226454
- US-A1- 2010 106 115
- US-A1- 2018 214 315

## Description

### TECHNICAL FIELD

This disclosure relates generally to medical treatment systems and, more particularly, but not by way of limitation, to dressings, systems, and methods for treating a tissue site with reduced pressure.

### BACKGROUND

Clinical studies and practice have shown that reducing pressure in proximity to a tissue site can augment and accelerate growth of new tissue at the tissue site. The applications of this phenomenon are numerous, but have proven particularly advantageous for treating wounds. Regardless of the etiology of a wound, whether trauma, surgery, or another cause, proper care of a wound is important to the outcome. Treatment of wounds or other tissue with reduced pressure may be commonly referred to as "negative-pressure therapy," but is also known by other names, including "negative-pressure wound therapy," "reduced-pressure therapy," "vacuum therapy," and "vacuum- assisted closure," for example. Negative-pressure therapy may provide a number of benefits, including migration of epithelial and subcutaneous tissues, improved blood flow, and micro- deformation of tissue at a wound site. Together, these benefits can increase development of granulation tissue and reduce healing times.

There is also widespread acceptance that cleansing a tissue site can be highly beneficial for new tissue growth. For example, a wound or a cavity can be washed out with a liquid solution for therapeutic purposes. These practices are commonly referred to as "irrigation" and "lavage" respectively. "Instillation" is another practice that generally refers to a process of slowly introducing fluid to a tissue site and leaving the fluid for a prescribed period of time before removing the fluid. For example, instillation of topical treatment solutions over a wound bed can be combined with negative-pressure therapy to further promote wound healing by loosening soluble contaminants in a wound bed and removing infectious material. As a result, soluble bacterial burden can be decreased, contaminants removed, and the wound cleansed.

While the clinical benefits of negative-pressure therapy and/or instillation are widely known, the cost and complexity of negative-pressure therapy and/or instillation can be a limiting factor in its application, and the development and operation of negative-pressure systems, components, and processes continues to present significant challenges to manufacturers, healthcare providers, and patients.
US2018/0214315 and WO 2019/226454 each disclose a negative pressure dressing with fluid instillation pathways through a manifold.

### SUMMARY

The invention is defined by the independent claims. A selection of optional features of the invention is set out in the dependent claims.

### BRIEF DESCRIPTION OF THE DRAWINGS

FIGURE 1 is a block diagram of an example embodiment of a therapy system and a dressing that can provide negative-pressure treatment and instillation in accordance with this specification;
FIGURE 2A is a plan view of an example embodiment of a tissue interface that can be associated with some embodiments of the system and dressing of FIGURE 1;
FIGURE 2B is a cross-section view of a portion of the example tissue interface of FIGURE 2A;
FIGURE 3A is an isometric view of another example embodiment of the tissue interface that can be associated with some embodiments of the system and dressing of FIGURE 1;
FIGURE 3B is a cross-section view of the example tissue interface of FIGURE 3A;
FIGURES 4A-4D illustrate via schematic diagrams an exemplary formation technique for forming a tissue interface similar to that of FIGURE 3A;
FIGURE 5 illustrates a schematic diagram of another exemplary formation technique for forming a tissue interface similar to FIGURE 2A;
FIGURE 6 is a schematic diagram, with a portion in cross-section, of an illustrative example embodiment of a tissue interface configured for instillation and negative-pressure therapy;
FIGURE 7A is a schematic diagram, with a portion in cross-section, of an illustrative example embodiment of an open-cavity, reduced-pressure treatment device and system;
FIGURE 7B is a schematic cross-section of a portion of the example treatment device of FIGURE 7A;
FIGURE 7C is a schematic cross-section of a portion of the example treatment device of FIGURE 7A taken along line 7C-7C;
FIGURE 7D is a schematic cross-section of a portion of the example system of FIGURE 7A;
FIGURE 8 is a schematic, isometric view of the example open-cavity, reduced- pressure treatment device of FIGURES 7A-7D;
FIGURE 9A is a schematic, plan view of another illustrative example embodiment of an open-cavity, reduced-pressure treatment device;
FIGURE 9B is a schematic, plan view of a portion of the example treatment device of FIGURE 9A;
FIGURE 9C is a schematic cross-section of a portion of the example treatment device of FIGURE 9B taken along line 9C-9C;
FIGURE 10 is an isometric view of an illustrative example embodiment of a manifold pad that can be associated with some embodiments of the system of FIGURE 7A; and
FIGURE 11 is a schematic diagram of a portion of an embodiment of a dressing and system similar to FIGURE 7A, illustrating additional details with regard to an exemplary embodiment of the manifold pad similar to FIGURE 2A.

### DETAILED DESCRIPTION OF ILLUSTRATIVE EMBODIMENTS

The following description of example embodiments enables a person skilled in the art to make and use the subject matter set forth in the appended claims. Certain details already known in the art may be omitted. Therefore, the following detailed description is illustrative and non-limiting.

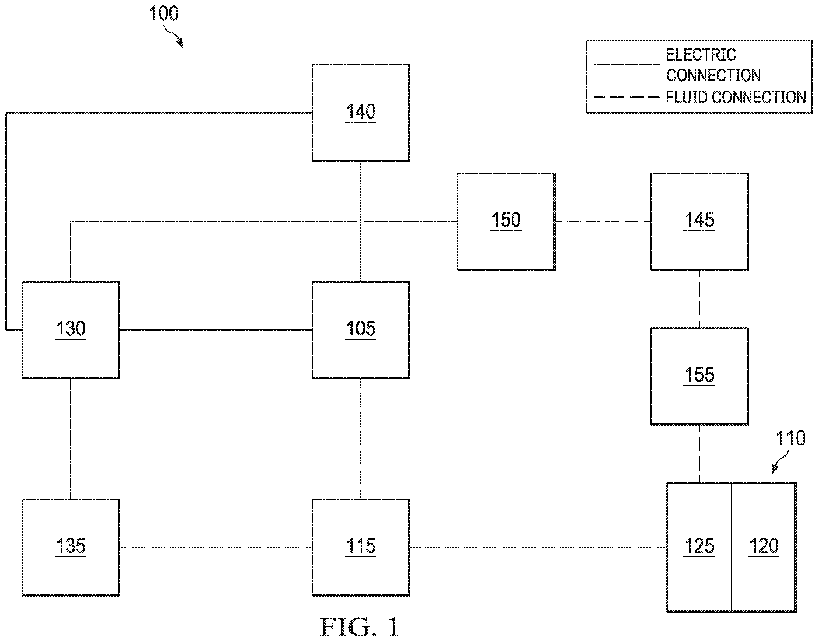

FIGURE 1 is a block diagram of an example embodiment of a therapy system 100 that can provide negative-pressure therapy with instillation of topical treatment solutions to a tissue site in accordance with this specification.

The term "tissue site" in this context broadly refers to a wound, defect, or other treatment target located on or within tissue, including but not limited to, a surface wound, bone tissue, adipose tissue, muscle tissue, neural tissue, dermal tissue, vascular tissue, connective tissue, cartilage, tendons, or ligaments. The term "tissue site" may also refer to areas of any tissue that are not necessarily wounded or defective, but are instead areas in which it may be desirable to add or promote the growth of additional tissue. For example, negative pressure may be applied to a tissue site to grow additional tissue that may be harvested and transplanted. A surface wound, as used herein, is a wound on a body that is exposed to the external environment, such as an injury or damage to the epidermis, dermis, and/or subcutaneous layers. Surface wounds may include ulcers or closed incisions, for example. A surface wound, as used herein, does not include wounds within an intra-abdominal cavity. A wound may include chronic, acute, traumatic, subacute, and dehisced wounds, partialthickness burns, ulcers (such as diabetic, pressure, or venous insufficiency ulcers), flaps, and grafts, for example.

The therapy system 100 may include a source or supply of negative pressure, such as a negative-pressure source 105, and one or more distribution components. A distribution component is preferably detachable and may be disposable, reusable, or recyclable. A dressing, such as a dressing 110, and a fluid container, such as a container 115, are examples of distribution components that may be associated with some examples of the therapy system 100. As illustrated in the example of FIGURE 1, the dressing 110 may include a tissue interface 120, a cover 125, or both in some embodiments.

A fluid conductor is another illustrative example of a distribution component. A "fluid conductor," in this context, broadly includes a tube, pipe, hose, conduit, or other structure with one or more lumina or open pathways adapted to convey a fluid between two ends. Typically, a tube is an elongated, cylindrical structure with some flexibility, but the geometry and rigidity may vary. Moreover, some fluid conductors may be molded into or otherwise integrally combined with other components. Distribution components may also include or comprise interfaces or fluid ports to facilitate coupling and de-coupling other components. In some embodiments, for example, a dressing interface may facilitate coupling a fluid conductor to the dressing 110. For example, such a dressing interface may be a SENSAT.R.A.C.^{™} Pad available from Kinetic Concepts, Inc. of San Antonio, Texas.

The therapy system 100 may also include a regulator or controller, such as a controller 130. Additionally, the therapy system 100 may include sensors to measure operating parameters and provide feedback signals to the controller 130 indicative of the operating parameters. As illustrated in FIGURE 1, for example, the therapy system 100 may include a first sensor 135 and a second sensor 140 coupled to the controller 130.

The therapy system 100 may also include a source of instillation solution. For example, a solution source 145 may be fluidly coupled to the dressing 110, as illustrated in the example embodiment of FIGURE 1. The solution source 145 may be fluidly coupled to a positive-pressure source, such as a positive-pressure source 150, a negative-pressure source such as the negative-pressure source 105, or both in some embodiments. A regulator, such as an instillation regulator 155, may also be fluidly coupled to the solution source 145 and the dressing 110 to ensure proper dosage of instillation solution (e.g. saline) to a tissue site. For example, the instillation regulator 155 may comprise a piston that can be pneumatically actuated by the negative-pressure source 105 to draw instillation solution from the solution source during a negative-pressure interval and to instill the solution to a dressing during a venting interval. Additionally or alternatively, the controller 130 may be coupled to the negative-pressure source 105, the positive-pressure source 150, or both, to control dosage of instillation solution to a tissue site. In some embodiments, the instillation regulator 155 may also be fluidly coupled to the negative-pressure source 105 through the dressing 110, as illustrated in the example of FIGURE 1.

Some components of the therapy system 100 may be housed within or used in conjunction with other components, such as sensors, processing units, alarm indicators, memory, databases, software, display devices, or user interfaces that further facilitate therapy. For example, in some embodiments, the negative-pressure source 105 may be combined with the controller 130, the solution source 145, and other components into a therapy unit.

In general, components of the therapy system 100 may be coupled directly or indirectly. For example, the negative-pressure source 105 may be directly coupled to the container 115 and may be indirectly coupled to the dressing 110 through the container 115. Coupling may include fluid, mechanical, thermal, electrical, or chemical coupling (such as a chemical bond), or some combination of coupling in some contexts. For example, the negative-pressure source 105 may be electrically coupled to the controller 130 and may be fluidly coupled to one or more distribution components to provide a fluid path to a tissue site. In some embodiments, components may also be coupled by virtue of physical proximity, being integral to a single structure, or being formed from the same piece of material.

A negative-pressure supply, such as the negative-pressure source 105, may be a reservoir of air at a negative pressure or may be a manual or electrically-powered device, such as a vacuum pump, a suction pump, a wall suction port available at many healthcare facilities, or a micro-pump, for example. "Negative pressure" or "reduced pressure" (which may be used interchangeably) generally refers to a pressure less than a local ambient pressure, such as the ambient pressure in a local environment external to a sealed therapeutic environment. In many cases, the local ambient pressure may also be the atmospheric pressure at which a tissue site is located. Alternatively, the pressure may be less than a hydrostatic pressure associated with tissue at the tissue site. Unless otherwise indicated, values of pressure stated herein are gauge pressures. References to increases in negative pressure typically refer to a decrease in absolute pressure, while decreases in negative pressure typically refer to an increase in absolute pressure. While the amount and nature of negative pressure provided by the negative-pressure source 105 may vary according to therapeutic requirements, the pressure is generally a low vacuum, also commonly referred to as a rough vacuum, between -5 mm Hg (-667 Pa) and -500 mm Hg (-66.7 kPa). Common therapeutic ranges are between -50 mm Hg (-6.7 kPa) and -300 mm Hg (-39.9 kPa), between -100 mm Hg and -200 mm Hg, or approximately -125 mm Hg.

The reduced pressure delivered may be constant, varied, patterned, or random, and may be delivered continuously or intermittently. Although the terms "vacuum" and "negative pressure" may be used to describe the pressure applied to the tissue site, the actual pressure applied to the tissue site may be more than the pressure normally associated with a complete vacuum. Consistent with the use herein, an increase in reduced pressure corresponds to a reduction in pressure (more negative relative to ambient pressure) and a decrease in reduced pressure corresponds to an increase in pressure (less negative relative to ambient pressure).

The container 115 is representative of a container, canister, pouch, or other storage component, which can be used to manage exudates and other fluids withdrawn from a tissue site. In many environments, a rigid container may be preferred or required for collecting, storing, and disposing of fluids. In other environments, fluids may be properly disposed of without rigid container storage, and a re-usable container could reduce waste and costs associated with negative-pressure therapy.

A controller, such as the controller 130, may be a microprocessor or computer programmed to operate one or more components of the therapy system 100, such as the negative-pressure source 105. In some embodiments, for example, the controller 130 may be a microcontroller, which generally comprises an integrated circuit containing a processor core and a memory programmed to directly or indirectly control one or more operating parameters of the therapy system 100. Operating parameters may include the power applied to the negative-pressure source 105, the pressure generated by the negative-pressure source 105, or the pressure distributed to the tissue interface 120, for example. The controller 130 is also preferably configured to receive one or more input signals, such as a feedback signal, and programmed to modify one or more operating parameters based on the input signals.

Sensors, such as the first sensor 135 and the second sensor 140, are generally known in the art as any apparatus operable to detect or measure a physical phenomenon or property, and generally provide a signal indicative of the phenomenon or property that is detected or measured. For example, the first sensor 135 and the second sensor 140 may be configured to measure one or more operating parameters of the therapy system 100. In some embodiments, the first sensor 135 may be a transducer configured to measure pressure in a pneumatic pathway and convert the measurement to a signal indicative of the pressure measured. In some embodiments, for example, the first sensor 135 may be a piezo-resistive strain gauge. The second sensor 140 may optionally measure operating parameters of the negative-pressure source 105, such as a voltage or current, in some embodiments. Preferably, the signals from the first sensor 135 and the second sensor 140 are suitable as an input signal to the controller 130, but some signal conditioning may be appropriate in some embodiments. For example, the signal may need to be filtered or amplified before it can be processed by the controller 130. Typically, the signal is an electrical signal, but may be represented in other forms, such as an optical signal.

The tissue interface 120 can be generally adapted to partially or fully contact a tissue site. The tissue interface 120 may take many forms, and may have many sizes, shapes, or thicknesses, depending on a variety of factors, such as the type of treatment being implemented or the nature and size of a tissue site. For example, the size and shape of the tissue interface 120 may be adapted to the contours of deep and irregular shaped tissue sites. Any or all of the surfaces of the tissue interface 120 may have an uneven, coarse, or jagged profile.

In some embodiments, the tissue interface 120 may include or may be a manifold. A manifold in this context may include a means for collecting or distributing fluid across or through the tissue interface 120 under pressure. For example, a manifold may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as fluid from a source of instillation solution, across a tissue site.

In some embodiments, the cover 125 may provide a bacterial barrier and protection from physical trauma. The cover 125 may also be constructed from a material that can reduce evaporative losses and provide a fluid seal between two components or two environments, such as between a therapeutic environment and a local external environment. The cover 125 may be, for example, an elastomeric film or membrane that can provide a seal adequate to maintain a negative pressure at a tissue site for a given negative-pressure source. Further, the cover 125 may be vapor permeable and/or liquid impermeable, thereby allowing vapor and inhibiting liquids from exiting a sealed space between the cover 125 and the tissue site. The cover 125 may have a high moisturevapor transmission rate (MVTR) in some applications. For example, the MVTR may be at least 250 grams per square meter per twenty-four hours in some embodiments, measured using an upright cup technique according to ASTM E96/E96M Upright Cup Method at 38°C and 10% relative humidity (RH). In some embodiments, the cover 125 may be a flexible, breathable film, membrane, or sheet having a high moisture vapor transfer rate (MVTR) of, for example, at least about 300g/m2 per 24 hours. In other embodiments, a low or no vapor transfer drape might be used. The cover 125 may comprise a range of medically suitable films having a thickness up to about 50 microns (µm). In some embodiments, an MVTR up to 5,000 grams per square meter per twenty-four hours may provide effective breathability and mechanical properties.

In some example embodiments, the cover 125 may be a polymer drape, such as a polyurethane film, that is permeable to water vapor but impermeable to liquid. Such drapes typically have a thickness in the range of 25-50 microns. For permeable materials, the permeability generally should be low enough that a desired negative pressure may be maintained. The cover 125 may be, for example, one or more of the following materials: polyurethane (PU), such as hydrophilic polyurethane; cellulosics; hydrophilic polyamides; polyvinyl alcohol; polyvinyl pyrrolidone; hydrophilic acrylics; silicones, such as hydrophilic silicone elastomers; natural rubbers; polyisoprene; styrene butadiene rubber; chloroprene rubber; polybutadiene; nitrile rubber; butyl rubber; ethylene propylene rubber; ethylene propylene diene monomer; chlorosulfonated polyethylene; polysulfide rubber; ethylene vinyl acetate (EVA); co-polyester; and polyether block polyamide copolymers. Such materials are commercially available as, for example, Tegaderm^{®} drape, commercially available from 3M Company, Minneapolis Minnesota; polyurethane (PU) drape; polyether block polyamide copolymer (PEBAX), for example from Arkema, France; and INSPIRE 2301 and INSPIRE 2327 polyurethane films, commercially available from Expopack Advanced Coatings, Wrexham, United Kingdom. In some embodiments, the cover 125 may comprise INSPIRE 2301 having an MVTR (upright cup technique) of 2600 g/m2/24 hours and a thickness of about 30 microns, or an MVTR (inverted cup technique) of 14400 g/m2/24 hours and a thickness of about 30 microns.

An attachment device may be used to attach the cover 125 to an attachment surface, such as undamaged epidermis, a gasket, or another cover. The attachment device may take many forms. For example, the attachment device may be a medically-acceptable, pressure-sensitive adhesive configured to bond the cover 125 to epidermis around a tissue site. In some embodiments, for example, some or all of the cover 125 may be coated with an adhesive, such as an acrylic adhesive, which may have a coating weight of about 25-65 grams per square meter (g.s.m.). Thicker adhesives, or combinations of adhesives, may be applied in some embodiments to improve the seal and reduce leaks. Other example embodiments of an attachment device may include a double-sided tape, paste, hydrocolloid, hydrogel, silicone gel, or organogel.

The solution source 145 may also be representative of a container, canister, pouch, bag, or other storage component, which can provide a solution for instillation therapy. Compositions of solutions may vary according to a prescribed therapy, but examples of solutions that may be suitable for some prescriptions include hypochlorite-based solutions, silver nitrate (0.5%), sulfurbased solutions, biguanides, cationic solutions, and isotonic solutions.

In operation, the tissue interface 120 may be placed within, over, on, or otherwise proximate to a tissue site. If the tissue site is a wound, for example, the tissue interface 120 may partially or completely fill the wound, or it may be placed over the wound. The cover 125 may be placed over the tissue interface 120 and sealed to an attachment surface near a tissue site. For example, the cover 125 may be sealed to undamaged epidermis peripheral to a tissue site. Thus, the dressing 110 can provide a sealed therapeutic environment proximate to a tissue site, substantially isolated from the external environment, and the negative-pressure source 105 can reduce pressure in the sealed therapeutic environment.

In general, exudate and other fluid flow toward lower pressure along a fluid path. Thus, the term "downstream" may refer to a location in a fluid path relatively closer to a source of negative pressure or further away from a source of positive pressure. Conversely, the term "upstream" may refer to a location further away from a source of negative pressure or closer to a source of positive pressure. Similarly, it may be convenient to describe certain features in terms of fluid "inlet" or "outlet" in such a frame of reference. This orientation is generally presumed for purposes of describing various features and components herein. However, the fluid path may also be reversed in some applications, such as by substituting a positive-pressure source for a negative-pressure source.

Negative pressure applied across the tissue site through the tissue interface 120 in the sealed therapeutic environment can induce macro-strain and micro-strain in the tissue site. Negative pressure can also remove exudate and other fluid from a tissue site, which can be collected in container 115.

In some embodiments, the controller 130 may receive and process data from one or more sensors, such as the first sensor 135. The controller 130 may also control the operation of one or more components of the therapy system 100 to manage the pressure delivered to the tissue interface 120. In some embodiments, controller 130 may include an input for receiving a desired target pressure and may be programmed for processing data relating to the setting and inputting of the target pressure to be applied to the tissue interface 120. In some example embodiments, the target pressure may be a fixed pressure value set by an operator as the target negative pressure desired for therapy at a tissue site and then provided as input to the controller 130. The target pressure may vary from tissue site to tissue site based on the type of tissue forming a tissue site, the type of injury or wound (if any), the medical condition of the patient, and the preference of the attending physician. After selecting a desired target pressure, the controller 130 can operate the negative-pressure source 105 in one or more control modes based on the target pressure and may receive feedback from one or more sensors to maintain the target pressure at the tissue interface 120.

In some embodiments, the controller 130 may have a continuous pressure mode, in which the negative-pressure source 105 is operated to provide a constant target negative pressure for the duration of treatment or until manually deactivated. Additionally or alternatively, the controller may have an intermittent pressure mode. For example, the controller 130 can operate the negative-pressure source 105 to cycle between a target pressure and atmospheric pressure. For example, the target pressure may be set at a value of 135 mmHg for a specified period of time (e.g., 5 min), followed by a specified period of time (e.g., 2 min) of deactivation. The cycle can be repeated by activating the negative-pressure source 105, which can form a square wave pattern between the target pressure and atmospheric pressure.

In some example embodiments, the increase in negative-pressure from ambient pressure to the target pressure may not be instantaneous. For example, the negative-pressure source 105 and the dressing 110 may have an initial rise time. The initial rise time may vary depending on the type of dressing and therapy equipment being used. For example, some therapy systems may increase negative pressure at a rate of about 20-30 mmHg/second, and other therapy systems may increase negative pressure at a rate of about 5-10 mmHg/second. If the therapy system 100 is operating in an intermittent mode, the repeating rise time may be a value substantially equal to the initial rise time.

In some example dynamic pressure control modes, the target pressure can vary with time. For example, the target pressure may vary in the form of a triangular waveform, varying between a negative pressure of 50 and 135 mmHg with a rise rate of negative pressure set at a rate of 25 mmHg/min. and a descent rate set at 25 mmHg/min. In other embodiments of the therapy system 100, the triangular waveform may vary between negative pressure of 25 and 135 mmHg with a rise rate of about 30 mmHg/min and a descent rate set at about 30 mmHg/min.

In some embodiments, the controller 130 may control or determine a variable target pressure in a dynamic pressure mode, and the variable target pressure may vary between a maximum and minimum pressure value that may be set as an input prescribed by an operator as the range of desired negative pressure. The variable target pressure may also be processed and controlled by the controller 130, which can vary the target pressure according to a predetermined waveform, such as a triangular waveform, a sine waveform, or a saw-tooth waveform. In some embodiments, the waveform may be set by an operator as the predetermined or time-varying negative pressure desired for therapy.

In some embodiments, the controller 130 may receive and process data, such as data related to instillation solution provided to the tissue interface 120. Such data may include the type of instillation solution prescribed by a clinician, the volume of fluid or solution to be instilled to a tissue site ("fill volume"), and the amount of time prescribed for leaving solution at a tissue site ("dwell time") before applying a negative pressure to the tissue site. The fill volume may be, for example, between 10 and 500 mL, and the dwell time may be between one second to 30 minutes. The controller 130 may also control the operation of one or more components of the therapy system 100 to instill solution. For example, the controller 130 may manage fluid distributed from the solution source 145 to the tissue interface 120. In some embodiments, fluid may be instilled to a tissue site by applying a negative pressure from the negative-pressure source 105 to reduce the pressure at the tissue site, drawing solution into the tissue interface 120. In some embodiments, solution may be instilled to a tissue site by applying a positive pressure from the positive-pressure source 150 to move solution from the solution source 145 to the tissue interface 120. Additionally or alternatively, the solution source 145 may be elevated to a height sufficient to allow gravity to move solution into the tissue interface 120.

The controller 130 may also control the fluid dynamics of instillation by providing a continuous flow of solution or an intermittent flow of solution. Negative pressure may be applied to provide either continuous flow or intermittent flow of solution. The application of negative pressure may be implemented to provide a continuous pressure mode of operation to achieve a continuous flow rate of instillation solution through the tissue interface 120, or it may be implemented to provide a dynamic pressure mode of operation to vary the flow rate of instillation solution through the tissue interface 120. Alternatively, the application of negative pressure may be implemented to provide an intermittent mode of operation to allow instillation solution to dwell at the tissue interface 120. In an intermittent mode, a specific fill volume and dwell time may be provided depending, for example, on the type of tissue site being treated and the type of dressing being utilized. After or during instillation of solution, negative-pressure treatment may be applied. The controller 130 may be utilized to select a mode of operation and the duration of the negative pressure treatment before commencing another instillation cycle.

Some dressings can be worn for a time period extending beyond seven days, which can be referred to as an extended wear time. Dressings for extended wear time can provide costsavings, time-efficiencies, and less trauma to a patient during dressing changes.

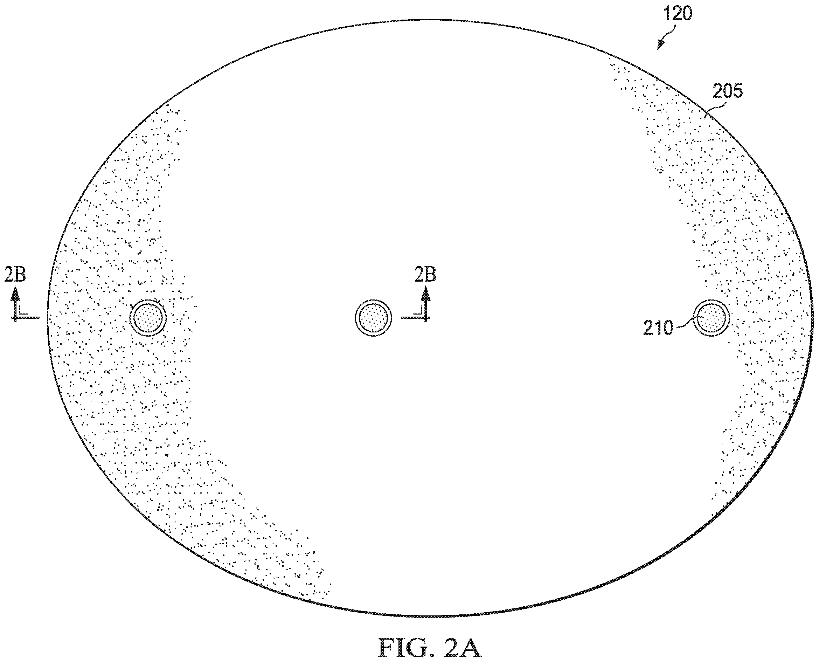

FIGURE 2A is a plan view of an example of the tissue interface 120 of FIGURE 1, illustrating additional details that may be associated with some embodiments. Herein, various examples of the tissue interface 120 are described that may be suitable for use with the dressing 110 and the therapy system 100. Further, features or elements of the tissue interface 120 described herein may be referred to as part of the therapy system 100 or the dressing 110 without reference to the tissue interface 120.

In some embodiments, the tissue interface 120 may include or may be a manifold, such as a manifold layer 205. The manifold layer 205 may provide a means for collecting or distributing fluid across the tissue interface 120 under pressure. For example, the manifold layer 205 may be adapted to receive negative pressure from a source and distribute negative pressure through multiple apertures across the tissue interface 120, which may have the effect of collecting fluid from across a tissue site and drawing the fluid toward the source. In some embodiments, the fluid path may be reversed or a secondary fluid path may be provided to facilitate delivering fluid, such as from a source of instillation solution, across the tissue interface 120.

In some illustrative embodiments, the pathways of the manifold layer 205 may be interconnected to improve distribution or collection of fluids. In some illustrative embodiments, the manifold layer 205 may be a porous material having interconnected fluid pathways. Examples of suitable porous material that comprise or can be adapted to form interconnected fluid pathways may include cellular foam, including open-cell foam such as reticulated foam; porous tissue collections; and other porous material such as gauze or felted mat that generally include pores, edges, and/or walls. Liquids, gels, and other foams may also include or be cured to include apertures and fluid pathways. In some embodiments, the manifold layer 205 may additionally or alternatively include projections that form interconnected fluid pathways. For example, the manifold layer 205 may be molded to provide surface projections that define interconnected fluid pathways.

In FIGURE 2A, the manifold layer 205 may be an open-cell foam having pore sizes that may vary according to needs of a prescribed therapy. For example, a foam having an average pore size in a range of 50-120 microns may be particularly suitable for some types of therapy. The tensile strength of the manifold layer 205 may also vary according to needs of a prescribed therapy. For example, the tensile strength of a foam may be increased for instillation of topical treatment solutions. In some embodiments, the manifold layer 205 may have a 25% compression load deflection of about 1.8 to about 2.8 pounds per square inch, and a 65% compression load deflection of about 2.2 to about 3.4 pounds per square inch (1 pound per square inch = 6.89476 kPa). In some embodiments, the manifold layer 205 may be a foam comprised of polyols such as polyester or polyether, isocyanate such as toluene diisocyanate, and polymerization modifiers such as amines and tin compounds. In some examples, the manifold layer 205 may be a reticulated polyurethane foam such as used in GRANUFOAM^{™} Dressing or V.A.C. VERAFLO^{™} Dressing, both available from KCI of San Antonio, Texas.

In some embodiments, the manifold layer 205 may be formed by a felting process. For example, the porous material may be a foam treated or modified to have a desired porosity or density. Any porous foam suitable for felting may be used, including the example foams mentioned herein, such as GRANUFOAM^{™} Dressing. In general, felting comprises a thermoforming process that permanently compresses a foam to increase the density of the foam while maintaining interconnected pathways. For example, in order to create felted foam, such as felted polyurethane, a foam blank of uncompressed material can be heated to an optimum forming temperature and then compressed.

Felting may be performed by any known methods, which may include applying heat and pressure to a porous material or foam material. Such methods may include compressing the porous material between one or more heated platens or dies (not shown) for a specified period of time and at a specified temperature. In some embodiments, the direction (e.g. axis) of compression may be along or parallel to the initial thickness of the foam blank of porous material. The felting process may alter certain properties of the original material, including pore shape and/or size, elasticity, density, and density distribution. For example, struts that define pores in the foam may be deformed during the felting process, resulting in flattened pore shapes. In some embodiments, the felting process may form the ovoidal or ellipsoidal pores or cells. The deformed struts can also decrease the elasticity of the foam. The density of the foam is generally increased by felting. In some embodiments, contact with hot-press platens in the felting process can also result in a density gradient in which the density is greater at the surface and the pores size is smaller at the surface.

A felted foam may be characterized by a firmness factor, which is indicative of the compression of the foam. The firmness factor of a felted foam can be specified as the ratio of original thickness to final thickness. A compressed or felted foam may have a firmness factor greater than 1. The degree of compression may affect the physical properties of the felted foam. For example, felted foam typically may have an increased effective density compared to a foam of the same material that is not felted. The felting process can also affect fluid-to-foam interactions. For example, as the density increases, compressibility or collapse may decrease in some directions. There is a general linear relationship between firmness level, density, pore size (or pores per inch) and compressibility. For example, foam found in a GRANUFOAM^{™} dressing that is felted to a firmness factor of 3 will show a three-fold density increase and compress to about a third of its original thickness under a given compression load. In some example embodiments, a firmness factor can range from about 2 to about 10. In some embodiments, the firmness factor of the manifold layer 205 felted foam may be greater than one and/or no more than approximately 5. For example, the firmness factor of the manifold layer 205 may be approximately 2-5, or approximately 3.

The period of time of compression may range from 10 minutes up to 24 hours, though the time period may be more or less depending on the specific type of porous material used. Further, in some examples, the temperature may range between 120°C to 260°C. Generally, the lower the temperature of the platen, the longer a porous material must be held in compression. After the specified time period has elapsed, the pressure and heat will form a felted structure or surface on or through the porous material or a portion of the porous material. The felted structure may be comparatively smoother than any unfinished or non-felted surface or portion of the porous material. Further, the pores in the felted structure may be smaller than the pores throughout any unfinished or non-felted surface or portion of the porous material. In some examples, the felted structure may be applied to all surfaces or portions of the porous material. Further, in some examples, the felted structure may extend into or through an entire thickness of the porous material such that the all of the porous material is felted. Typically, the entire manifold layer 205 may be felted to the same firmness factor throughout, although in other embodiments, the firmness factor may vary throughout the manifold layer 205.

In some embodiments, the felted foam for the manifold layer 205 may be formed from a foam blank of open cell foam, such as polyurethane foam, having an initial (e.g. pre-felted) density of approximately 1.3-1.6 lb/ft³ (1 lb/ft³ = 16.0185 kg/m³), a free volume of approximately 90% or more, an average number of pores per inch of approximately 40-50 (1 inch = 2.54 cm), an average pore size of approximately 400-600 micron, a 25% compression load deflection of at least 0.35 pounds per square inch, and/or a 65% compression load deflection of at least 0.43 pounds or square inch. The felted foam may have an axis (e.g. direction) of compression. In some embodiments, the foam blank may be compressed and/or felted in the direction of the axis of compression (e.g. parallel to the axis of compression), to form the foam with ovoidal pores. In some embodiments, the minor axis of the ovoidal pores may be substantially parallel to the axis of compression and/or the major axis of the ovoidal pores may be substantially perpendicular to the axis of compression. In some embodiments, the felted foam may be compressed (e.g. felted) to have a firmness factor of greater than 1 to no more than 5, or in other embodiments, from 2-7, 3-7, 2-5, 3-5, 2-3, or 5-7.

In some embodiments, the manifold layer 205 may comprise or consist essentially of an open-cell foam having a free volume in a range of about 13% to about 45%, a density of about 2.6-11.2 lb/ft³, about 80-350 pores per inch on average (e.g., as measured in the direction/axis of compression), and/or average pore size of about 57-300 micron (e.g., as measured in the direction/axis of compression); in some embodiments, a foam blank (for example, similar to that described above) may be felted to obtain foam with these characteristics. In some embodiments, the manifold layer 205 may comprise or consist essentially of an open-cell foam having a free volume in a range of about 18% to about 45%, a density of about 2.6-8.0 lb/ft³, about 80-250 pores per inch on average (e.g., as measured in the direction/axis of compression), and/or average pore size of about 80-300 micron (e.g., as measured in the direction/axis of compression); in some embodiments, a foam blank (for example, similar to that described above) may be felted to obtain foam with these characteristics. In some embodiments, the manifold layer 205 may comprise or consist essentially of an open-cell foam having a free volume in a range of about 18% to about 30%, a density of about 3.9-8.0 lb/ft³, about 120-250 pores per inch on average (e.g., as measured in the direction/axis of compression), and/or average pore size of about 80-200 micron (e.g., as measured in the direction/axis of compression); in some embodiments, a foam blank (for example, similar to that described above) may be felted to obtain foam with these characteristics. In some embodiments, the manifold layer 205 may comprise or consist essentially of an open-cell foam having a free volume in a range of about 30% to about 45%, a density of about 2.6-4.8 lb/ft³, about 80-150 pores per inch on average (e.g., as measured in the direction/axis of compression), and/or average pore size of about 133-300 micron (e.g., as measured in the direction/axis of compression); in some embodiments, a foam blank (for example, similar to that described above) may be felted to obtain foam with these characteristics. In some embodiments, the manifold layer 205 may comprise or consist essentially of an open-cell foam having a free volume in a range of about 30-33%, a density of about 3.9-4.8 lb/ft³, about 120-150 pores per inch on average (e.g., as measured in the direction/axis of compression), and/or average pore size of about 133-200 micron (e.g., as measured in the direction/axis of compression); in some embodiments, a foam blank (for example, similar to that described above) may be felted to obtain foam with these characteristics.

In some embodiments, the manifold layer 205 may be a closed-cell foam such as, for example, those manufactured by Zotefoams, Inc. of Walton, Kentucky, U.S.A., including the Azote, Plastazote, Evazote, Supazote, and Zotek grades. Such closed-cell foams may be manufactured by extruding polymer sheets or blocks and crosslinking through high energy radiation. Suitable polymers may include low and high density polyolefins and copolymers with vinyl acetate, fluoropolymers, polyamides, and PEBAX. The polymer sheets may then be softened under heat and exposed to high pressure nitrogen gas which dissolves in the polymer. After cooling, the polymer sheets may be heated again and exposed to subatmospheric pressure, resulting in expansion and formation of the closed-cell foam. In some embodiments, the closed-cell foam may be thermoformed. Thermoforming the foam involves embossing patterns and structures, such as channels, into the surfaces of the foam in order to translate what would otherwise be non-manifolding material into a structure that will manifold pressure and fluid along the channels.

In some embodiments, one or more suitable foam blanks may be used for forming the manifold layer 205. The foam blanks may be open cell foam, felted foam, closed cell foam, or another foam as described herein. However, the properties of any of these foam blanks may have about 200 to about 400 pores per inch on average, a density of about 6.5 to about 12.8 lb/ft³, a free volume of about 90% or more, an average pore size in a range of about 50 to about 120 microns, and/or a 25% compression load deflection of at least 1.5 pounds per square inch (1 square inch = 6.4516 cm²). In some embodiments, the foam blank(s) may have a thickness greater than 8 millimeters, for example 8-16 millimeters.

FIGURES 2A-2B show an exemplary tissue interface 120 having an example manifold layer 205, illustrating addition details that may be associated with some embodiments of the system of Figure 1. In some embodiments, the manifold layer 205 may comprise open-cell foam, which may be configured for negative-pressure therapy, and at least one skinned channel 210 may extend into the open-cell foam. In some embodiments, the open-cell foam may be configured to distribute negative-pressure from a first surface 220 of the manifold layer 205 to a second surface 225 of the manifold layer 205, and thereby to the tissue site (e.g. through the porous structure of the open-cell foam). Generally, the at least one skinned channel 210 may extend from a first surface 220 (e.g. configured to face away from the tissue site) into the open-cell foam, and may be configured to distribute liquid, such as instillation solution, from the first surface 220 through the manifold layer 205. For example, in FIGURES 2A-2B, a plurality of skinned channels 210 may extend through the open-cell-foam, from the first surface 220 to the second surface 225 (with the second surface 225 configured to face towards the tissue site and/or towards a treatment device of the dressing). In some embodiments, a first opening 222 of the skinned channel 210 may be disposed on the first surface 220 of the manifold layer 205, allowing fluid communication through the first surface 220 into the corresponding channel 210 within the manifold layer 205. In some embodiments, a second opening 227 of the skinned channel 210 may be disposed on the second surface 225 of the manifold layer 205, allowing fluid communication from the channel 210 through the second surface 225 and out of the manifold layer 205.

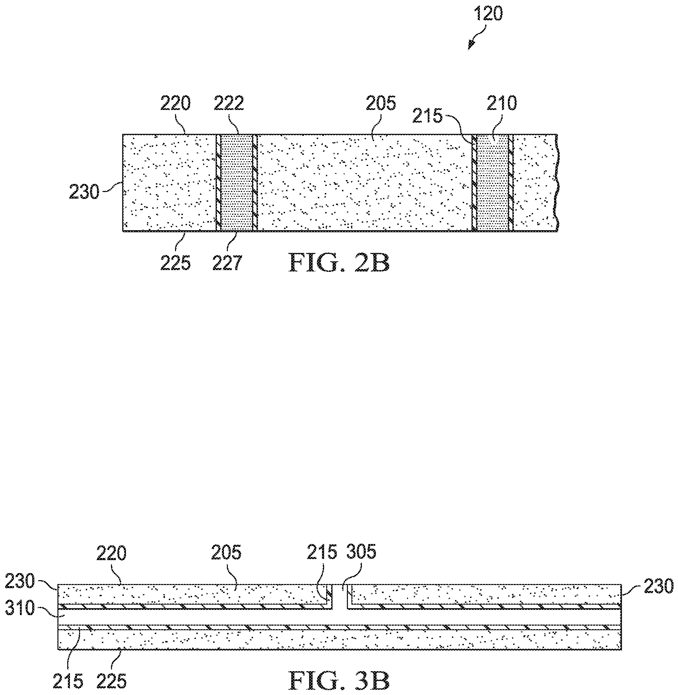

In some embodiments, such as shown in FIGURE 2B, the at least one skinned channel 210 may comprise a skin 215. The skin 215 may be disposed along an entire perimeter and an entire length of each channel 210, from the first opening 222 or end of the channel 210 to the second opening 227 or end of the channel 210. In some embodiments, the skin 215 may be disposed along the entire inner surface or entire interface of each channel 210 with the open-cell foam, for example, sealing the interface. In some embodiments, the skin 215 may be more liquid impermeable than the open-cell foam itself. (e.g. less permeable to liquid than the open-cell foam). For example, the skin 215 may be substantially impermeable to liquid, such as instillation solution, so that during instillation, instillation solution from within the skinned channels 210 will not enter, flow into, penetrate, or soak into the open-cell foam (e.g. liquid, such as instillation solution, will not pass through the skin 215). In some embodiments, the skin 215 may be substantially non-porous and/or closed-cell. In some embodiments, the skin may be configured to fluidly isolate the at least one skinned channel 210 from other portions of the manifold 205, such as the open-cell foam, as the channel 210 passes through the manifold layer 205.

In some embodiments, the skin 215 may be integrally formed with the open-cell foam, for example at the interface of the channel 210 and the open-cell foam. For example, the skin 215 may include or may be a liquid impermeable surface formed on a portion of the open-cell foam (e.g. the interface of the channel 210 and the open-cell foam). In some embodiments, the skin 215 may be formed by a portion of the open-cell foam (e.g. at interface between open-cell foam and channels 210), for example with the skin 215 comprising or consisting essentially of a substrate material of the open-cell foam. The substrate material may be less liquid permeable than the remained of the open-cell foam or, in some embodiments, substantially liquid impermeable. In some embodiments, the substrate material may be substantially closed-cell. In some embodiments, the substrate material may be substantially non-porous.

In some embodiments, the at least one skinned channel 210 may be configured for instillation (e.g. to allow transport of instillation solution through the open-cell foam without allowing instillation solution to substantially exit the skinned channel 210 through the skin 215 and enter the foam during instillation). In some embodiments, the at least one skinned channel 210 may have a diameter of at least 2.3 mm. In some embodiments, each skinned channel 210 may have a diameter greater than approximately 1/8 inch and/or less than approximately ¾ inch. For example, each of the plurality of skinned channels 210 may have a diameter of approximately ¼ - 1/2 inch. In some embodiments, each skinned channel 210 may extend with an approximately constant diameter (e.g. the diameter may be approximately uniform along its length). In some embodiments, all of the skinned channels 210 may have approximately the same diameter. In some embodiments, the surface area of the first opening of the at least one channel 210 may be at less than 5% of the surface area of the first surface 220. In some embodiments, the volume of the channels 210 as a whole may comprise no more than 5% of the total volume of the open-cell foam.

In some embodiments, the open-cell foam may be or comprise felted foam, and the at least one skinned channel 210 (e.g. the skin 215 of the channel 210) may be formed during the felting process for forming the felted foam. For example, the skin 215 of the skinned channel 210 may be formed during the felting process as an integral portion of the foam (e.g. an integral substrate material) at the interface between the channel 210 and the felted open-cell foam. In some embodiments, the skin 215 may comprise a substantially smaller cell structure than the felted open-cell foam, may be significantly less liquid permeable than the felted open-cell foam, and/or may be substantially liquid impermeable. In some embodiments, the felted foam may be configured to maintain a sufficiently open porous cell structure to allow for effective negative-pressure therapy therethrough (e.g. with the porous structure of the felted foam providing the fluid flowpath for negative-pressure distribution). In some embodiments, the felted foam may comprise a firmness factor greater than one and/or no more than about 5 (e.g. approximately 2-5). For example, the felted foam may have a firmness factor of approximately 3. In some embodiments, the felted foam may have a compression axis substantially perpendicular to the at least one skinned channel 210 (e.g. has been compressed in a direction perpendicular to the longitudinal centerline of at least one skinned channel 210). In some embodiments, the open-cell foam (e.g. the felted foam) and/or the skin 215 may provide sufficient support so that the skinned channels 210 do not completely collapse during negative pressure therapy and/or remain open during instillation and/or when in place on the tissue site.

As shown in FIGURE 2B, the manifold layer 205 may further comprise the first surface 220 configured to face away from the tissue site and/or towards the sealing member, the second surface 225 configured to face towards the tissue site and/or the treatment device, and a thickness of the open-cell foam between the first surface 220 and the second surface 225. In some embodiments, at least one skinned channel 210 may extend between the first surface 220 and the second surface 225. For example, at least one skinned channel 210 may comprise a vertical channel 305 configured to extend substantially from the first surface 220 to the second surface 225. In some embodiments, a plurality of the skinned channels 210 or all of the skinned channels 210 may extend from the first surface 220 to the second surface 225. In some embodiments, the plurality of skinned channels 210 may be configured to distribute instillation fluid from the first surface 220 to the second surface 225.

In some embodiments, the at least one skinned channel 210 may form a first fluid pathway through the manifold layer 205, and the porous open-cell structure of the open-cell foam (e.g. the felted foam) may form a second fluid pathway through the manifold layer 205. In some embodiments, the first fluid pathway and the second fluid pathway through the manifold layer 205 may be distinct and fluidly separated and/or isolated (e.g. by the skin 215 of the channels 210), so there can be no substantial migration of liquid from the instillation channels 210 through the skin 215 of the channel walls to the foam cell structure, or vice versa. In some embodiments, the channels 210 may be configured so that liquid within each channel 210 can only exit through the second opening 227 in fluid communication with the treatment device and/or tissue site. For example, the first fluid pathway may be configured only for instillation (e.g. allowing instillation through the channels 210 to bypass the open-cell foam and move through the manifold layer 205 independently of the second fluid pathway), and/or the second fluid pathway may be configured only for negative-pressure therapy (e.g. allowing negative-pressure therapy to occur exclusively through the porous structure of the open-cell foam, bypassing the skinned channels 210). In other embodiments, the first fluid pathway may be configured for instillation and negative-pressure therapy, and the second fluid pathway may only be configured for negative-pressure therapy. In some embodiments, the first fluid pathway may be configured to be fluidly coupled to an instillation solution source (e.g. by instillation interface). For example, the skinned channels 210 each may comprise an instillation opening 222 in the first surface 220, and the instillation opening 222 may be configured to be in fluid communication with (e.g. attachment to) an instillation interface leading to the instillation solution source.

In some embodiments, the manifold layer 205 may form a manifold pad, which may be configured for use with a treatment device. In some embodiments, a manifold pad may be used atop the treatment device, with the second surface 225 of the manifold pad facing, contacting and/or in fluid communication with the treatment device.

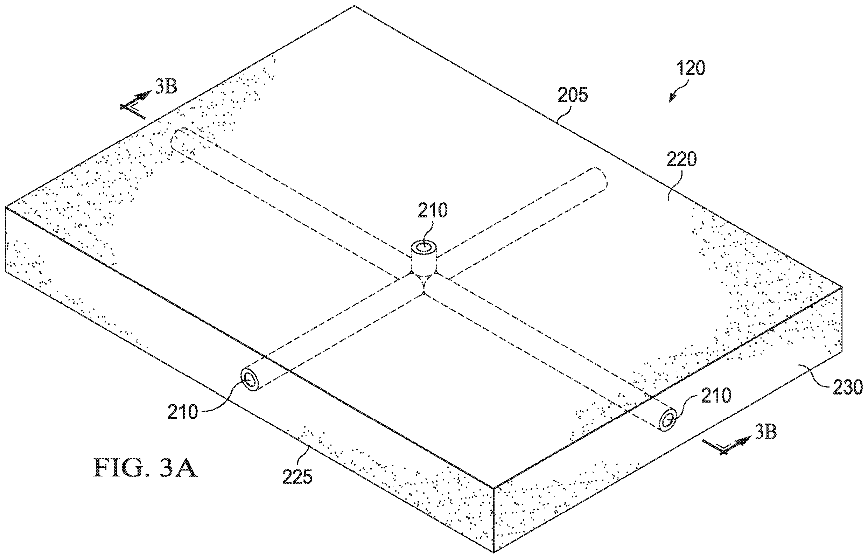

FIGURES 3A-3B show another example embodiment of the tissue interface that can be associated with some embodiments of the system and dressing of FIGURE 1, illustrating additional details that may be associate with some embodiments. The tissue interface of FIGURE 3A may be similar to that of FIGURE 2A, while further including one or more skinned channels 210 that do not extend from the first surface 220. In some embodiments, the open-cell foam may comprise perimeter sidewalls 230 extending substantially parallel to the thickness (e.g. substantially perpendicular to the first surface 220 and/or the second surface 225), and at least one skinned channel 210 may extend between perimeter sidewalls 230. In some embodiments, at least one channel 210 may extend substantially horizontally and/or perpendicular to the thickness. In some embodiments, at least one skinned channel 210 may extend between opposing perimeter sidewalls 230 (e.g. which are substantially opposite each other).

As shown in FIGURES 3A-3B, the plurality of skinned channels 210 may comprise at least one vertical channel 305 and at least one horizontal channel 310. In some embodiments, the plurality of channels 210 may comprise a plurality of horizontal channels 310. In some embodiments, at least one of the horizontal channels 310 may extend at least from the vertical channel 305 to one of the perimeter sidewalls 230. In some embodiments, the at least one vertical channel 305 may be in fluid communication with the at least one horizontal skinned channel. For example, the vertical channel 305 may extend from the first surface 220 to the horizontal channel 310 and/or each horizontal channel 310 may be in fluid communication with at least one vertical channel 305. In some embodiments, the horizontal channels 310 may extend approximately parallel to the first surface 220 and/or second surface 225. In some embodiments, the horizontal channels 310 may extend approximately perpendicular to the thickness of the manifold layer 205. In some embodiments, the horizontal channels 310 may be located approximately in a plane that is in proximity to the midpoint of the thickness (e.g. approximately halfway between the first surface 220 and the second surface 225). In some embodiments, the plurality of channels 210 may be configured to jointly distribute instillation solution from the first surface 220 of the manifold layer 205 to the perimeter of the manifold layer 205 (e.g. to a plurality of perimeter sidewalls 230) and/or to the second surface 225. While the channels 210 shown in FIGURES 2A-3B are straight, in other embodiments the channels 210 may not be straight (e.g. the channels 210 may be curved or may form other shapes).

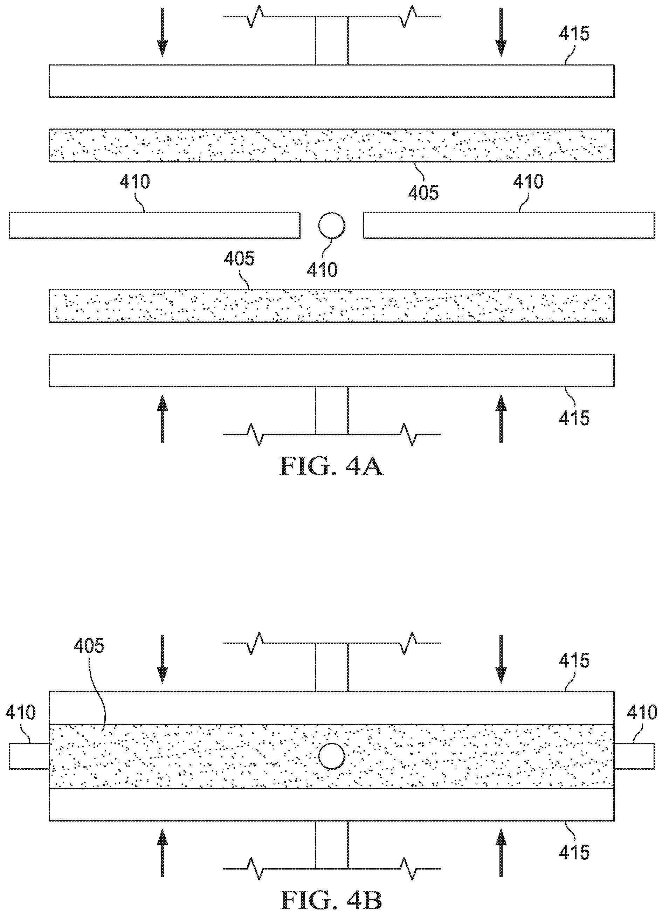

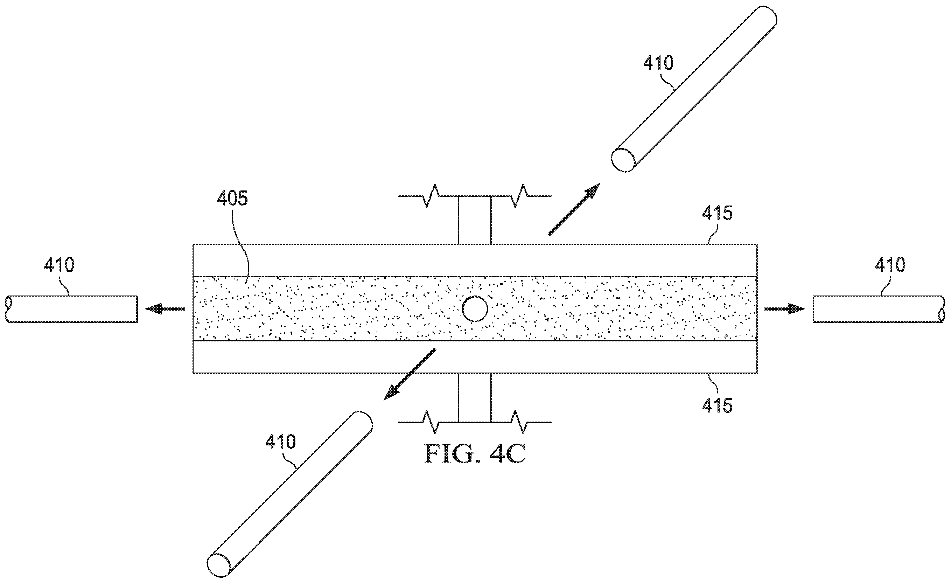

FIGURES 4A-4D illustrate a schematic diagram of an exemplary formation technique for forming a tissue interface similar to that of FIGURE 3A. For example, in some method of formation embodiments, a manifold layer 205 or pad configured for negative-pressure therapy and instillation may be formed by a process that may comprise the following steps: providing open-cell foam; disposing one or more heating cores 410 (configured to form channels 210) within the foam; heating the cores 410; and felting the open-cell foam. In some embodiments, felting the open-cell foam may comprise heating and compressing the foam simultaneously, as shown in FIGURE 4B for example. Some embodiments may further comprise removing the cores 410 from the felted foam (see for example, FIGURE 4C). In some embodiments, felting the open-cell foam while heating the cores 410 may form skinned channels 210, which may have a skin 215 that is substantially liquid impermeable and integrally formed with the open-cell foam. In some embodiments, disposing one or more heating cores 410 may comprise disposing the one or more heating cores 410 at locations corresponding to desired locations for skinned channels 210. In some embodiments, the heating cores 410 may be heated to approximately the same temperature as used felting the foam (e.g. during the felting process).

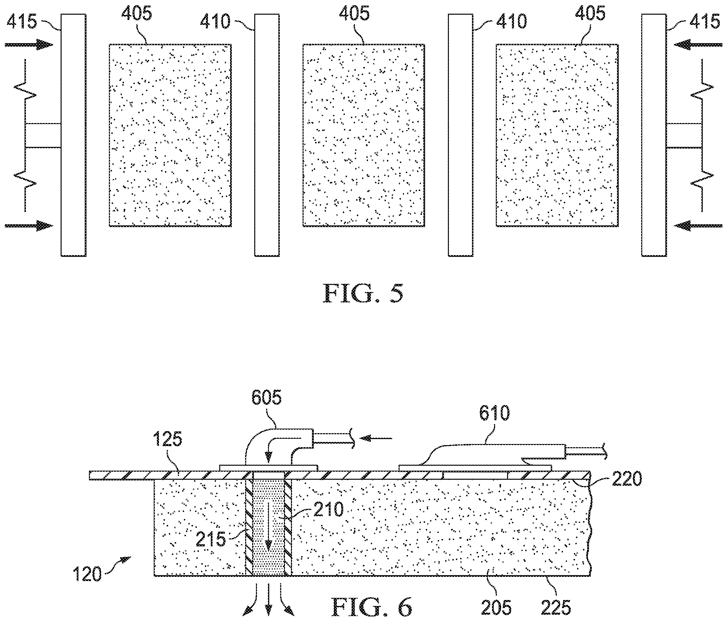

In some embodiments, providing the open-cell foam may comprise providing two or more open-cell foam blanks 405; and disposing the heating cores 410 within the foam may comprise disposing the heating cores 410 between the foam blanks 405 (e.g. blocks, sheets of foam), as shown in FIGURE 4A for example. In some embodiments, the process of felting the open-cell foam may join the two or more foam blanks 405 into a unitary whole (e.g. simultaneous with forming felted foam with increased density). For example, the heating cores 410 may be positioned between two foam blanks 405. In some embodiments, as shown in FIGURE 4A, one foam blank 405 may be positioned above the heating cores 410, and one foam blank 405 may be positioned below the heating cores 410 (and the heating cores 410 may be oriented substantially horizontally). Alternatively, as shown in FIGURE 5, one foam blank 405 may be positioned to the left of each heating core 410, and one foam blank 405 may be positioned to the right of each heating core 410 (and the heating cores 410 may be oriented substantially vertically). In other embodiments (not shown), providing the open-cell foam may comprise providing a single foam blank 405; and disposing the heating cores 410 within the foam may comprise forming (e.g. drilling) holes in the foam blank 405 and inserting the heating cores 410 into the holes within the foam.

In some embodiments, felting the open-cell foam may comprise compressing and heating the foam. In some embodiments, felting the open-cell foam may further comprise providing one or more felting platens 415 configured to felt the foam blanks 405 (e.g. by heating of the platens 415 and compressing the open-cell foam between the heated platens 415). For example, felting the open-cell foam may comprise disposing the open-cell foam between the platens 415; heating the platens 415; and compressing the open-cell foam between the platens 415 (e.g. by forcing the platens closer together while the open-cell foam is between the platens). In some embodiments, the foam may be felted/compressed to a firmness factor greater than 1 and/or less than about 5 (e.g. a firmness factor of approximately 2-5, or approximately 3). In some embodiments, felting the foam may comprise compressing the foam approximately perpendicular to the heating cores 410 (e.g. the axis of compression may be approximately perpendicular to the longitudinal axis of the cores 410). In some embodiments, felting the foam may comprise compressing the foam approximately parallel to the thickness of the manifold pad (e.g. the axis of compression may be approximately parallel to the thickness of the manifold pad).

In some embodiments, felting the foam may comprise compressing the foam approximately parallel to the heating cores 410 (e.g. axis of compression may be approximately parallel to the longitudinal axis of the cores 410). For example, the heating cores 410 may be configured with a length that corresponds approximately to the thickness of the manifold pad (after compression and heating), allowing the parallel compression to occur without damaging the heating cores 410. In some embodiments, compression during felting may occur until the platens 415 contact the heating cores 410. In other embodiments, the platens 415 may comprise holes corresponding to the heating cores 410 and configured to ensure that the heating cores 410 are not crushed by the platens 415 (e.g. allowing the heating cores 410 to slide through the holes during compression). In some embodiments, felting the foam may comprise compressing the foam approximately perpendicular to the thickness of the manifold pad (e.g. the axis of compression may be approximately perpendicular to the thickness of the manifold pad), as shown for example in FIGURE 5.

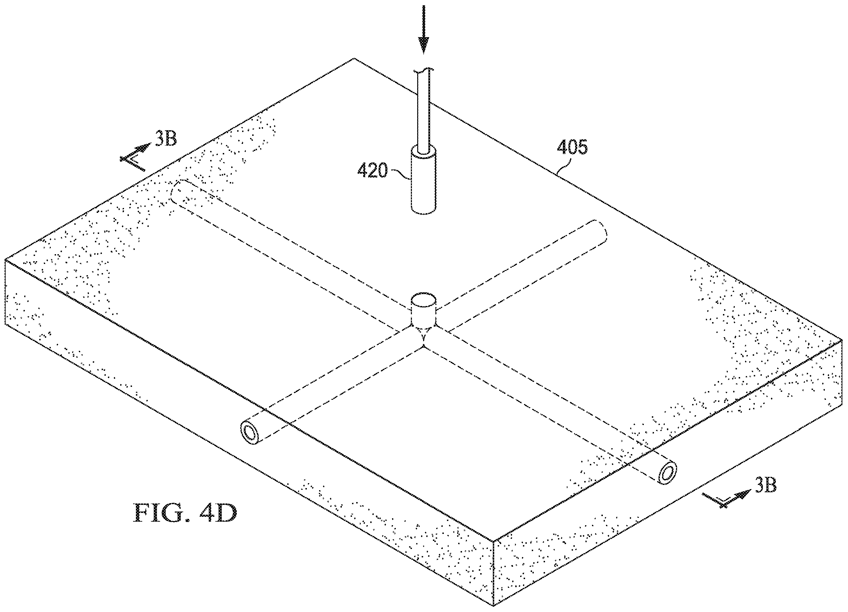

In some embodiments, the skinned channels 210 may extend horizontally (e.g. approximately parallel to the first surface 220 and/or the second surface 225, or approximately perpendicular to the thickness of the foam) and/or vertically (e.g. approximately parallel to the thickness and/or approximately perpendicular to the first surface 220 and/or the second surface 225) through the open-cell (e.g. felted) foam. Some embodiments may further comprise cutting a core channel (e.g. using a core bore 420) or other cutting or drilling element, to form channels and/or to fluidly couple the first surface 220 of the foam to the channels 210 (e.g. horizontal channels 310) formed by the heating cores 410, as shown in FIGURE 4D. In some embodiments, cutting a core channel may comprise heating the cutting or drilling element (e.g. core bore 420) while cutting into the foam. Alternatively, method embodiments may further comprise disposing a vertical heating core 410 within the first foam blank 405, wherein the vertical heating core 410 may extend from the first surface 220 to one or more of the horizontal heating cores 410.

While not shown in FIGURES 4A-D, in alternate embodiments of the method of formation the step of providing open-cell foam may comprise providing a single foam blank 405 and forming (e.g. drilling) holes in the foam blank 405 configured to receive the heating cores 410 for formation of the skinned channels 210. In some embodiments, disposing one or more heating cores 410 (configured to form channels 210) within the foam may comprise sliding the heating cores 410 into the holes in the foam blank 405. In other embodiments, the heating cores 410 may be integral to and/or may extend from inner surfaces of the heating platens 415 (e.g. as spikes or other projections), and disposing the heating cores 410 in the foam blank 405 and/or forming the skinned channels 210 may be accomplished as the heated cores 410 are heated while the platens 415 compress the foam blank 405 during felting (e.g. with the heating cores 410 piercing and extending into the foam blank 405 during the felting process).

In other alternate embodiments (not shown), the skin 215 may be formed as a film skin on a channel surface of the manifold layer 205 as part of a manufacturing process associated with the manifold layer 205. For example, a pre-polymer mixture configured to produce an open-cell foam may be placed or injected into a mold to form at least part of the manifold layer 205. In some embodiments, the pre-polymer mixture may include ingredients, such as isocyanate and polyol, for forming a polyurethane foam when mixed with water. Additional examples may include ingredients for forming a polyethylene foam when mixed with low-boiling-point liquids or pressurized gases, and accordingly the pre-polymer mixture may comprise molten polyethylene and low-boiling- point liquids such as fluorocarbons, pressurized gasses such as nitrogen, and/or chemical blowing agents such as citric acid and carbonate or bicarbonate salts. As the ingredients of the mixture react within the mold, a foam may be generated and a film may also develop on one or more surfaces of the foam. For example, as the foam contacts one or more cores (which may be disposed in the mold and configured to form the skinned channels 210) and/or walls of the mold, the relative temperature of the portion, or surface, of the foam contacting the wall of the mold may be reduced, thereby retarding or stopping the reaction of ingredients forming the foam. As a result, an integral film skin may be formed on the surfaces of the foam contacting the wall of the mold and/or cores, with the integral film skin being a portion of foam having a very high density, for example, a much higher density than the remainder of the generated foam. In some instances, the integral film skin may have a sufficiently high density to essentially be free of pores. In some examples where the foam is a polyurethane foam, the integral film skin may be or form a polyurethane elastomer. The integral film skin may provide the skin 215 as described herein, with the step of forming the skinned channels occurring simultaneously with forming and/or providing the open-cell foam. In some embodiments, perimeter portions of the molded foam may be removed to form the manifold layer 205. For example, the film skin may be removed (e.g. cut) from at least the first and second surfaces to expose the open-cell foam structure at those surfaces.

Further, in other examples, the skin 215 may be a surface of the manifold layer 205 along the channels 210 that has been treated to have liquid impermeable properties. In such an example, the manifold layer 205 foam interfacing the channels 210 may be treated by various methods including, without limitation, the application of a coating or a felting process.

FIGURE 5 illustrates a schematic diagram of another exemplary formation technique, which may be similar to that shown in FIGURES 4A-4D, for forming a tissue interface similar to FIGURE 2A. In Figure 5, one or more vertical skinned channels may be formed during the felting process. In FIGURE 5, the vertical channels 305 may extend from the first surface 220 to the second surface 225. In some method embodiments, steps from FIGURE 5 may be combined with steps from FIGURES 4A-4D, for example to form both vertical and horizontal channels 310 in the manifold pad using heating cores 410 during the felting of foam. In some embodiments, each horizontal channel 310 may be in fluid communication with at least one vertical channel 305.

FIGURE 6 is a schematic diagram of a portion in cross-section of an illustrative example embodiment of a system configured for instillation and negative-pressure therapy, illustrating additional details that may be associated with some embodiments. In some embodiments, the manifold layer 205, with one or more skinned channels 210, may form a manifold pad and/or tissue interface 120. The first surface 220 of the manifold layer 205 may be configured to face the cover 125, while the second surface 225 may be configured to face the tissue site and/or a treatment device. The one or more skinned channels 210 may be configured to be in fluid communication with the solution source 145, through the cover 125. For example, for each vertical channel, there may be an instillation interface 605 configured to be in fluid communication with the vertical channel (e.g. at the first surface 220). In some embodiments, only some (e.g. not all) of the vertical channels may be in fluid communication with an instillation interface 605. For example, medical personnel may determine which of the provided vertical channels to use for instillation, and may choose to make use of all or only a portion of the instillation channels 210. This may provide medical personnel with flexibility to determine the appropriate number and/or location of instillation channels 210 to use for a particular patient. In some embodiments, the instillation interface 605 may be configured to fluidly communicate and/or couple only with opening of the channels 210 on the first surface 220 of the manifold layer 205.

In some embodiments, each vertical channel may correspond to an opening in the cover 125, and each instillation interface 605 may be configured to sealingly attach to the cover 125 over the corresponding opening. The one or more instillation interface 605 may be in fluid communication with the instillation solution source 145, such that instillation may occur by fluid coupling of the solution source to the vertical channels via the instillation interface 605 (e.g. through the cover 125, via corresponding opening). In some embodiments, a negative-pressure interface 610 may be configured to fluidly couple the negative-pressure source 105 to the open-cell foam of the manifold layer 205, through the cover 125. For example, an opening in the cover 125 may allow the negative-pressure interface 610 to be in fluid communication with the porous open-cell foam structure of the manifold layer 205 (e.g. at the first surface 220). In some embodiments, the negative-pressure interface 610 may be configured to sealingly attach to the cover 125 over the corresponding opening. In some embodiments, the negative-pressure interface 610 may not contact and/or be in fluid communication with any skinned channels 210. This configuration may provide for two separate (e.g. fluidly isolated) flowpaths or fluid pathways through the manifold layer 205, for example a first instillation flowpath comprising the channels 210, and a second negative-pressure flowpath comprising the porous structure of the open-cell foam. The skin 215 of the skinned channels 210 may significantly limit or substantially prevent liquid flow from the channels 210 into the open-cell foam though the skin 215 walls of the channels 210. For example, the skin 215 may be configured to fluidly isolate the at least one skinned channel (e.g. the plurality of skinned channels) 210 from the open-cell foam as the channels 210 pass through the manifold layer 205. In some embodiments, both the instillation interface 605 and the negative-pressure interface 610 may be simultaneously attached to the dressing and/or in fluid communication with the channels 210 and the open-cell foam respectively.

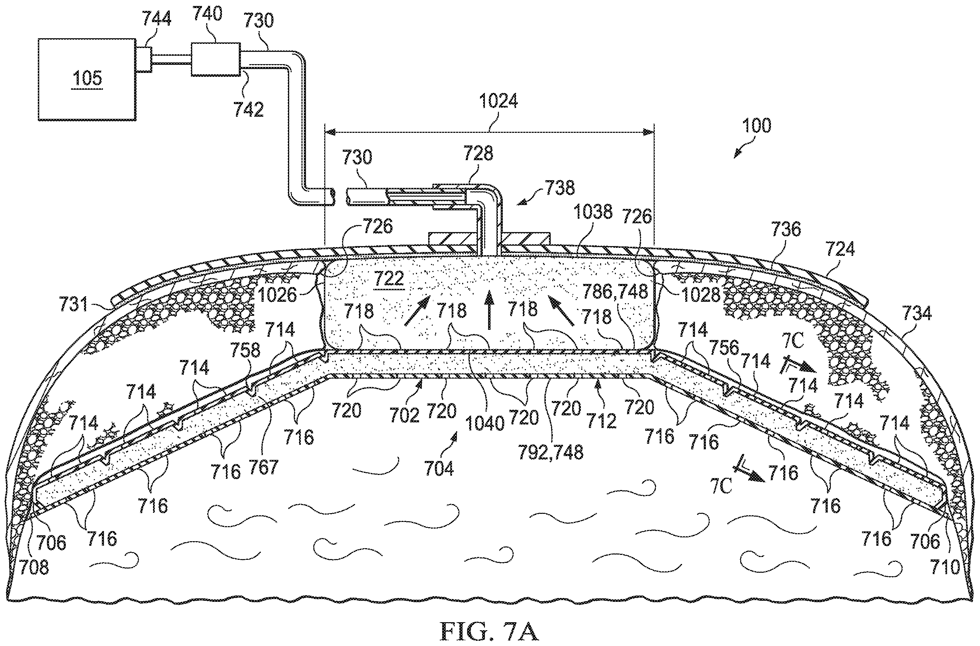

Referring to FIGURES 7A-7D, an illustrative embodiment of a therapy system 100, such as an open-cavity, reduced-pressure system, and a treatment device 702 is presented. The open-cavity reduced-pressure therapy system 100 and the treatment device 702 are for treating a tissue site 704 of a patient. The tissue site 704 may be the bodily tissue of any human, animal, or other organism, including bone tissue, adipose tissue, muscle tissue, dermal tissue, tissue, connective tissue, cartilage, tendons, ligaments, or any other tissue. In this illustrative embodiment, the tissue site 704 includes tissue in a body cavity, and in particular the abdominal cavity, and includes the abdominal contents or tissue that is proximate the abdominal cavity. Treatment of the tissue site 704 may include removal of fluids, e.g., exudate or ascites, protection of the abdominal cavity, or reduced-pressure therapy.

As shown, the treatment device 702 is disposed within the abdominal cavity of the patient to treat the tissue site 704. In some embodiments, the treatment device 702 may be configured to channel fluid radially under applied negative pressure and/or instillation. In some embodiments, the treatment deice 702 may comprise a film and/or a treatment manifold. The treatment device 702 of FIGURES 7A-D includes a plurality of elongate members, such as encapsulated leg members 706, that are supported by the abdominal contents, which make up a surface on which the plurality of leg members 706 are placed. One or more of the plurality of encapsulated leg members 706 may be placed in or proximate to a first paracolic gutter 708, and one or more of the plurality of encapsulated leg members 706 may be placed in or proximate to a second paracolic gutter 710. The plurality of encapsulated leg members 706 may be coupled to a central connection member 712 or central hub, and there may be fluid communication between the plurality of encapsulated leg members 706 and the central connection member 712. The plurality of encapsulated leg members 706 and/or the central connection member 712 may be formed with fenestrations 714, 716, 718, 720 that allow fluids in the abdominal cavity to pass through. The fenestrations 714, 716, 718, 720 may take any shape, e.g., circular apertures, rectangular openings, polygons, etc., but are presented in this illustrative embodiment as slits, or linear cuts. One or more fenestrations 714, 716, 718, 720 might be omitted in alternative embodiments.

A manifold pad 722, which may comprise or be similar to the manifold layer 205, distributes reduced pressure to the treatment device 702. A sealing member 724, which may be similar to the cover 125, provides a pneumatic seal over body-cavity opening 726. One or more skin closure devices may be placed on a patient's epidermis 734. Reduced pressure is delivered to the manifold pad 722 through a reduced-pressure interface 728 (which may be similar to the negative-pressure interface 610), which is coupled to a reduced-pressure delivery conduit 730. A negative-pressure source 105 delivers reduced pressure to the reduced-pressure delivery conduit 730.

The reduced pressure may be applied to the tissue site 704 to help promote removal of ascites, exudates, or other fluids from the tissue site 704. In some instances, reduced pressure may be applied to stimulate the growth of additional tissue. In some instances, only fluid removal may be desired. In the case of a wound at the tissue site 704, the growth of granulation tissue, removal of exudates, or removal of bacteria may help to promote healing of the wound. In the situation of a nonwounded or non-defective tissue, reduced pressure may be used in some instances to promote the growth of tissue that may be harvested and transplanted to another tissue site. Reduced pressure may initially generate fluid flow in the manifold pad 722, the reduced-pressure conduit 730, and proximate the tissue site 704. As the hydrostatic pressure around the tissue site 704 approaches the desired reduced pressure, the flow may subside, and the reduced pressure may be maintained.

The manifold pad 722 is proximate the central connection member 712. The manifold pad 722 may take many forms. The term "manifold" as used herein generally refers to a substance or structure that is provided to assist in applying reduced pressure to, delivering fluids to, or removing fluids from the tissue site 704. The manifold pad 722 typically includes a plurality of flow pathways that distribute the fluids provided to and removed from the tissue site 704 around the manifold pad 722 and through the central connection member 712. In one illustrative embodiment, the flow pathways are interconnected to improve distribution of fluids provided or removed from the tissue site 704. The manifold pad 722 may be a biocompatible material that is capable of being placed in contact with the tissue site 704 and distributing reduced pressure to the tissue site 704. In one embodiment, the manifold pad 722 is a porous foam and includes a plurality of interconnected cells or pores that act as flow pathways.

The sealing member 724 is configured to be placed over the body-cavity opening 726 and may be formed from any material capable of providing a pneumatic seal or fluid seal adequate for the open-cavity, reduced-pressure system 100 to hold a reduced pressure at the tissue site 704. The sealing member 724 may be a cover, similar to cover 125, that is used to secure the manifold pad 722 on the central connection member 712. The sealing member 724 may be impermeable or semipermeable. The sealing member 724 is capable of maintaining reduced pressure at the tissue site 704 after installation of the sealing member 724 over the body-cavity opening 726. In some embodiments, the sealing member 724 may be a flexible over-drape or film formed from a silicone-based compound, acrylic, hydrogel or hydrogel-forming material, or any other biocompatible material that includes the impermeability or permeability characteristics as desired for applying reduced pressure to the tissue site 704.

In some embodiments, the sealing member 724 may be vapor permeable and/or liquid impermeable, thereby allowing vapor and inhibiting liquids from exiting a sealed space between the sealing member 724 and the tissue site 704. In some embodiments, the sealing member 724 may be a flexible, breathable film, membrane, or sheet having a high moisture vapor transfer rate (MVTR) of, for example, at least about 250g/m2 per 24 hours. In other embodiments, a low or no vapor transfer drape might be used. The sealing member 724 may comprise a range of medically suitable films having a thickness up to about 50 microns (µm).

The sealing member 724 may further include an attachment means 731 to secure the sealing member 724 to the patient's epidermis 734. In some embodiments, the attachment means 731 may be similar to the attachment device associated with cover 125. The attachment means 731 may take many forms; for example, an adhesive layer 736 may be positioned along a perimeter of the sealing member 724 or any portion of the sealing member 724 to provide, directly or indirectly, the pneumatic seal with the patient's epidermis 734. The adhesive layer 736 might also be pre-applied to the sealing member 724 and covered with a releasable backing, or member (not shown), that is removed at the time of application.

The reduced-pressure interface 728 may be, as one example, a port or connector 738, which permits the passage of fluid from the manifold pad 722 to the reduced-pressure delivery conduit 730 and vice versa. For example, fluid collected from the tissue site 704 using the manifold pad 722 and the treatment device 702 may enter the reduced-pressure delivery conduit 730 via the connector 738. In another embodiment, the open-cavity, reduced-pressure system 100 may omit the connector 738 and the reduced-pressure delivery conduit 730 may be inserted directly into the sealing member 724 and into the manifold pad 722. The reduced-pressure delivery conduit 730 may be a medical conduit or tubing or any other means for transportating a reduced pressure and fluid. The reduced-pressure delivery conduit 730 may be a multi-lumen member for readily delivering reduced pressure and removing fluids. In one embodiment, the reduced-pressure delivery conduit 730 is a two-lumen conduit with one lumen for reduced pressure and liquid transport and one lumen for communicating pressure to a pressure sensor.

Reduced pressure is generated and supplied to the reduced-pressure delivery conduit 730 by the negative-pressure source 105. A wide range of reduced pressures may be generated or supplied by the negative-pressure source 105. In one embodiment, the range may include -50 to -300 mm Hg, and in another embodiment, the range may include -100 mm Hg to -200 mm Hg. In one illustrative embodiment, the negative-pressure source 105 includes preset selectors for -100 mm Hg, - 125 mm Hg, and -150 mm Hg. The negative-pressure source 105 may also include a number of alarms, such as a blockage alarm, a leakage alarm, or a battery-low alarm.

The negative-pressure source 105 may be any suitable device for providing reduced pressure, such as, for example, a vacuum pump, wall suction, hand pump, manual pump, electronic pump, micro-pump, piezoelectric pump, diaphragm pump, a portable source, wall source, a unit for abdominal cavities, or other source. The negative-pressure source 105 may include control circuitry and sensors, such as a pressure sensor, that may be configured to monitor reduced pressure at the tissue site 704. The negative-pressure source 105 may also be configured to control the amount of reduced pressure from the negative-pressure source 105 being applied to the tissue site 704 according to a user input and a reduced-pressure feedback signal received from the tissue site 704. The negative-pressure source 105 may selectively deliver a constant pressure, varied pressure, intermittent pressure, or continuous pressure. The fluid removed from the cavity through the reduced-pressure delivery conduit 730 could be as much as 5 liters or more per day.

A number of different devices, e.g., device 740, may be added to a medial portion 142 of the reduced-pressure delivery conduit 730. For example, the device 740 might be a fluid reservoir, or canister collection member, a pressure-feedback device, a volume detection system, a blood detection system, an infection detection system, a filter, a port with a filter, a flow monitoring system, a temperature monitoring system, etc. Multiple devices 740 might be included. Some of these devices, e.g., the fluid collection member, may be formed integral to the negative-pressure source 105. For example, a reduced-pressure port 744 on the negative-pressure source 105 may include a filter member (not shown) that includes one or more filters and may include a hydrophobic filter that prevents liquid from entering an interior space of the negative-pressure source 105.

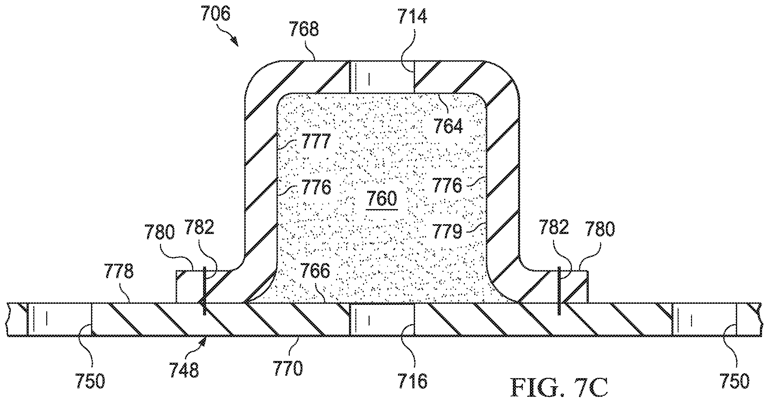

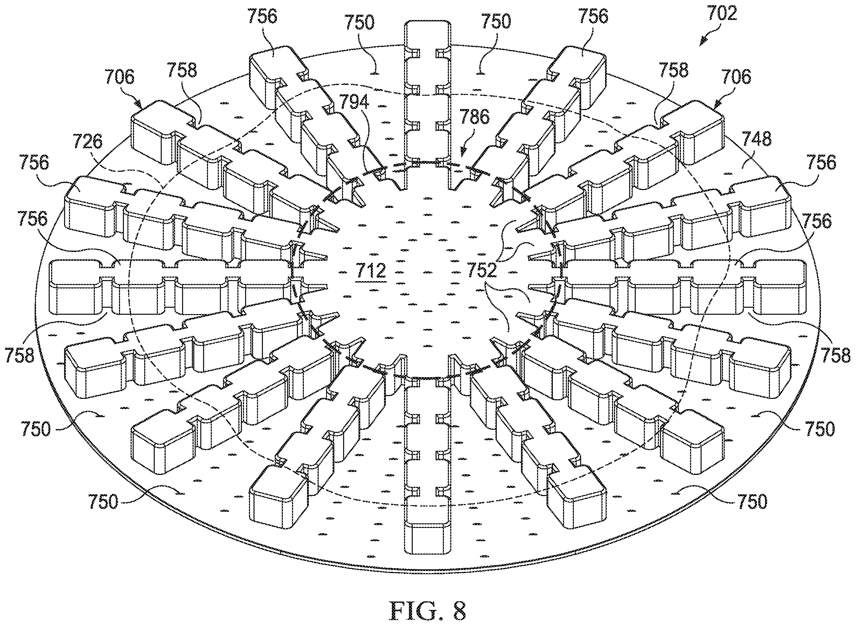

Referring now to FIGURES 7A, 7C, and 8, the treatment device 702 can include a non-adherent drape 748. The non-adherent drape 748 may be formed of a non-adherent material that inhibits tissue adhesion to the non-adherent drape 748. In one embodiment, the non-adherent drape 748 is formed from a breathable polyurethane film. The non-adherent drape 748 can include a plurality of openings, apertures, or fenestrations 750. The fenestrations can take a variety of shapes, such as circular openings, rectangular openings, polygon-shaped openings, etc., but are shown in FIGURE 8 as slits, or linear cuts. Depending on the particular application of device 702, the desired fluid flow and/or pressure delivery, or other system parameters, the fenestrations can have different sizes.

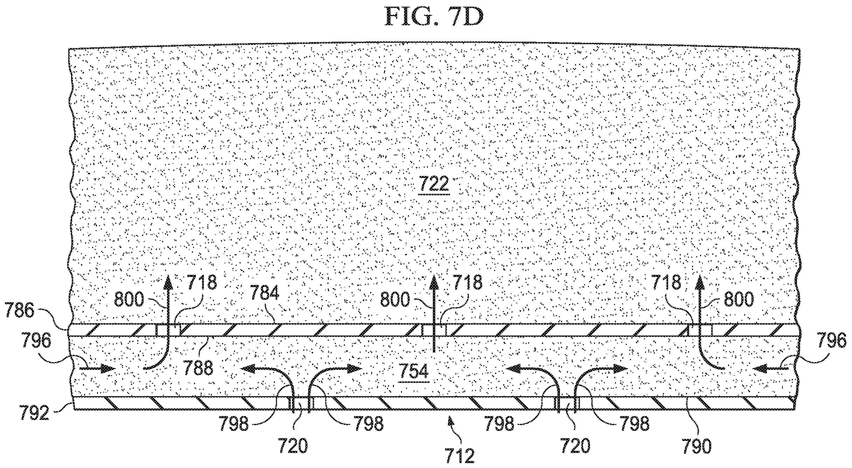

Referring to FIGURES 7A, 7D, and 8, the treatment device 702 includes the central connection member 712 to which the plurality of encapsulated leg members 706 are coupled. The central connection member 712 includes a connection manifold member 754 that is encapsulated by a first connection encapsulation member 786, which may be referred to as a first non-adherent drape 786, and a second connection encapsulation member 792, which may be referred to as a second non-adherent drape 792. A portion of the central connection member 712 can fluidly couple at leg coupling areas 752 to permit fluid communication between the central connection member 712 and the plurality of encapsulated leg members 706. The first and the second connection encapsulation member or non-adherent drape 786, 792 may be defined by a single piece of material or, as illustrated, more than one sheet of material. For example, the non-adherent drape 748 may be used as a single sheet or may include the first non-adherent drape 786 and the second non-adherent drape 792.

The central connection member 712, as discussed above, can fluidly communicate with manifold pad 722. In one aspect, fenestrations 718, similar to the fenestrations discussed above, can permit fluid communication. Additionally, or alternatively, a portion or portions of the first connection encapsulation member or non-adherent drape 786 can be exposed to manifold pad 722.

Referring again to FIGURES 7A-7D, each of the plurality of encapsulated leg members 706 can include a leg manifold member 760, which may be a single manifold member that runs between leg modules 756 and/or central connection member 712, or individual manifold components. The leg manifold member 760 is disposed within an interior portion 762 of each of the encapsulated leg members 706. Each leg manifold member 760 has a first side 764 and a second, inward-facing (patient-facing) side 766.

In some embodiments, the connection manifold member 754 and one or more of the leg manifold members 760 extending from the connection manifold member 754 may form a treatment manifold 767. The treatment manifold 767 including the leg manifold members 760 and the connection manifold member 754 may comprise or be formed of a foam or a manifold material similar or analogous to the manifold materials described herein for the manifold pad 722.

The non-adherent drape 748 may surround the treatment manifold 767. For example, the non-adherent drape 748 may be coupled around the leg manifold members 760 and the connection manifold member 754 to provide the plurality of encapsulated leg members 706 in fluid communication with the central connection member 712. In some embodiments, the non-adherent drape 748 may include the first non-adherent drape 786 and the second non-adherent drape 792 and the treatment manifold 767 may be positioned as a layer between the first non-adherent drape 786 and the second non-adherent drape 792. The first non-adherent drape 786 may be coupled to the second non-adherent drape 792 to provide the plurality of encapsulated leg members 706 in fluid communication with the central connection member 712.

In one embodiment, one or more of the plurality of leg manifold members 760 can have different material properties or structures. For example, different flow rates may be desired in different encapsulated leg members 706. In one aspect, different manifold materials or manifold properties, different manifold sizes, manifold compression, the use flow restricting material structures, and/or or valves can provide different flow rates of fluid through the encapsulated leg members and/or central connection member.

In one aspect, a first leg encapsulating member 768, which can be formed with fenestrations 714, is disposed on the first side 764 of the leg manifold member 760. A second leg encapsulating member 770, which can include fenestrations 716, is disposed on the second, inward-facing side 766 of the leg manifold member 760. The first leg encapsulating member 768 and the second leg encapsulating member 770 may be a portion of the non-adherent drape 748. In some embodiments, the first leg encapsulating member 768 may be a portion of the first non-adherent drape 786 and the second leg encapsulating member 770 may be a portion of the second non-adherent drape 792. In some embodiments, the encapsulated leg members 706 can be mated with one another, for example, via the drape 748. In some embodiments, the encapsulated leg members 706 can be independently movable with respect to one another with the exception of their proximal end adjacent to the central connection member 712. For example, the encapsulated leg members 706 need not be connected to one another. In another embodiment, a portion of the material connecting the encapsulated leg members 706, e.g., the non-adherent drape 748 between adjacent encapsulated leg members 706, is expandable (e.g., a stretchable, flexible, deformable, and/or elastic material) and permits movement of individual encapsulated leg members 706 relative to one another.

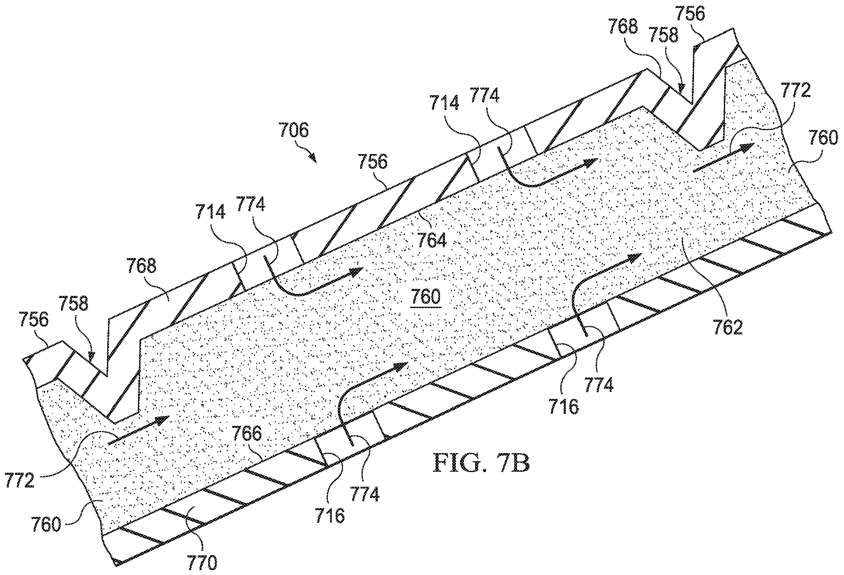

As shown in the longitudinal cross section of FIGURE 7B by arrows 772, fluid can flow from leg modules 756 towards the central connection member 712. As shown by arrows 774, the fluid is able to enter fenestrations 714 and 716 and flow into the leg manifold member 760 and then flow toward the central connection member 712 as represented by arrows 772.

In plan view, the encapsulated leg members 706 may take a number of different shapes, such as elongate shapes, rectangular, elliptical, etc. In one aspect, the encapsulated leg members 706 may include leg modules 756. Adjacent leg modules 756 are fluidly coupled to each other and have a manipulation zone 758 between them. In one aspect, the manipulation zone includes a weakened or perforated area to facilitate sizing of the device. For example, a clinician can cut through a leg module to size the device. By pulling on the partially cut leg module the manifold can be torn away at the next manipulation zone. In one aspect, the recessed shape of the manipulation zone 758 can inhibit accidental removal of additional leg modules. Additionally, or alternatively, the outer portion of the leg modules can be fixed to the device to inhibit unwanted manifold removal.

The encapsulated leg members 706 may also have various dimensions. If the longer dimension, e.g., lengthwise or longitudinal dimension, of the encapsulated leg 706 is L1 and the width is W1, then the aspect ratio is given by L1/W1. The aspect ratio may be 8.0, 7.0, 6.0, 5.0, 4.0, 3.0, 2.0 or any number in between. Moreover, other aspect ratios are possible. Generally, the width W1 of the encapsulated leg member will be greater than a width W2 of the central connection member 712, i.e., W2 > W1. For example, in one illustrative embodiment, the encapsulated leg members 706 are approximately 270 mm long, 60 mm wide (W1), and 10 mm thick, and the central connection member has a width parallel to the first width (W1) of about 130 mm (W2). Thus, in that illustrative example, the aspect ratio of the encapsulated leg 706 is approximately (270/60) or 4.5. In this same illustrative embodiment, the manipulation zones 758 have a width of about 10 mm.

Referring to FIGURE 7C, a lateral cross section of a portion of the encapsulated leg member 706 is presented. As before, it can be seen that the first side 764 of the leg manifold member 760 is covered with the first leg encapsulating member 768, and that the second, inward-facing side 766 of the leg manifold member 760 is covered by the second leg encapsulating member 770, which in this instance is a portion of the non-adherent drape 748. Thus, in this illustrative embodiment, the fenestrations 716 may be some of the plurality of fenestrations 750 in the non-adherent drape 748. In this illustrative embodiment, peripheral edges 776 of the leg manifold member 760 are also covered by a portion of the first leg encapsulating member 768. The peripheral edges 776 include a first lateral edge 777 and a second lateral edge 779. The first leg encapsulating member 768 covers the first side 764 and the peripheral edges 776 and extends onto a first surface 778 of the non-adherent drape 748 and forms extensions 780. The extensions 780 have been coupled to the second leg encapsulating member 770 by welds 782. The first leg encapsulating member 768 may, however, be coupled to the second leg encapsulating member 770 using any known technique, including welding (e.g., ultrasonic or RF welding), bonding, adhesives, cements, etc.

In some embodiments, at least a portion of a longitudinal length of at least one of the encapsulated leg members 706 is configured to contact the tissue site 704. Further, in some embodiments, the fenestrations 716 may be positioned along the longitudinal length of at least one of the encapsulated leg members 760.

Referring again to FIGURE 7D and FIGURE 8, the central connection member 712 includes the connection manifold member 754 that is encapsulated within the first connection encapsulation member 786, which has fenestrations 718. The first connection encapsulation member 786 is disposed on a first side 788 of the connection manifold member 754. The second connection encapsulation member 792 is disposed on a second, inward-facing side 790 of the connection manifold member 754. The second connection encapsulation member 792 is formed with fenestrations 720. The first connection encapsulation member 786 has a peripheral zone or edge 794 as shown in FIGURE 8. In a similar fashion, the second connection encapsulation member 792 has a peripheral zone or edge (not explicitly shown) that lines up with the peripheral edge 794. The peripheral edge 794 of the first connection encapsulation member 786 is coupled to peripheral edge of the second connection encapsulation member 792, except at the leg coupling areas 752 in order to allow fluid within the plurality of encapsulated leg members 706 to flow into the connection manifold member 754 as suggested by arrows 796 in FIGURE 7D. Fluid may also enter directly into the connection manifold member 754 by flowing through fenestrations 720 as suggested by arrows 798. The manifold pad 722 is disposed proximate to the first connection encapsulation member 786, and when a reduced pressure is applied to the manifold pad 722, the reduced pressure causes fluid to flow from the connection manifold member 754 through fenestrations 718 and into the manifold pad 722 as suggested by arrows 800. The fluid continues to flow in the direction of the reduced-pressure interface 728 through which the fluid is removed to the reduced-pressure delivery conduit 730.

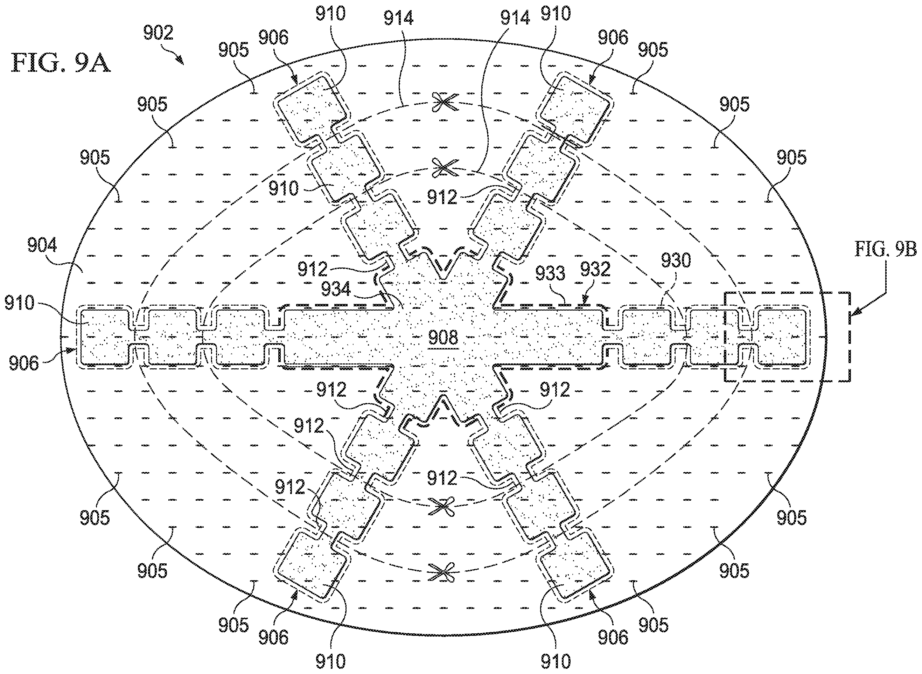

Referring to FIGURES 7A-7D and 8, in operation, the illustrative open-cavity, reduced-pressure system 100 may be used by first sizing the treatment device 702 as will be explained further below in connection with FIGURE 9A. The non-adherent drape 748 with the plurality of encapsulated leg members 706 is disposed within the abdominal cavity through the body-cavity opening 726 and is distributed against the abdominal contents; this may include placing at least one encapsulated leg member 706 in or proximate the first paracolic gutter 708, the second paracolic gutter 710, or behind the liver, etc. Once the treatment device 702 has been distributed, the manifold pad 722 is placed adjacent a first side 784 of the first connection encapsulation member 786. The sealing member 724 may then be applied over the body-cavity opening 726 to provide a pneumatic seal over the body-cavity opening 726.

In addition to the sealing member 724, the body-cavity opening 726 may be further closed or reinforced using mechanical closing means, e.g., staples or tension cords (such as Dermaclose or ABRA surgical skin closure), or using a reduced-pressure closure system. The sealing member 724 may be applied in a number of ways, but according to one illustrative embodiment, the releasable backing member that is on the adhesive layer 736 of the sealing member 724 is removed and then the sealing member 724 is placed against the patient's epidermis 734 about the body-cavity opening 726. The reduced-pressure interface 728, such as port 738, is then coupled or attached to the sealing member 724 such that reduced pressure can be delivered by the interface 728, through the sealing member 724, and to the manifold pad 722 and the central connection member 754. The reduced-pressure delivery conduit 730 is fluidly coupled to the reduced-pressure interface 728 and to the reduced-pressure port 744 on the negative-pressure source 105.

The negative-pressure source 105 is activated and thereby provides reduced pressure into the reduced-pressure delivery conduit 730, which delivers the reduced pressure to the reduced-pressure interface 728 and into the manifold pad 722 and the central connection member 754. The manifold pad 722 distributes the reduced pressure and draws fluid through fenestrations 718 from the connection manifold member 754. The connection manifold member 754 draws fluid from the abdominal cavity through fenestrations 720 and pulls fluid from the plurality of encapsulated leg members 706 as suggested by arrows 796. Fluid from the abdominal cavity flows into the plurality of encapsulated leg members 706 through fenestrations 714 on the first leg encapsulating member 768 and through fenestrations 716 on the second leg encapsulating member 770 and then flows through the leg as suggested by arrows 772 towards the connection manifold member 754. The fluid then flows through the manifold pad 722, the reduced-pressure interface 728, and into the reduced-pressure delivery conduit 730.

Referring now to FIGURES 9A-9C, another illustrative embodiment of an open-cavity, reduced-pressure treatment device 902 is presented. The open-cavity, reduced-pressure treatment device 902 is analogous in most respects to the treatment device 702 of FIGURES 7A-7D. The open-cavity, reduced-pressure treatment device 902 has a non-adherent drape 904, a plurality of encapsulated leg members 906, and a central connection member 908. In this particular illustrative embodiment, the non-adherent drape 904 is formed generally with an oval or arcuate shape. The non-adherent drape 904 is formed with a plurality of fenestrations 905. The non-adherent drape 904 forms the second leg encapsulating member (see by analogy second leg encapsulating member 770 in FIGURE 7B) and the second connection encapsulation member (see by analogy 792 in FIGURE 7D). As such, the plurality of fenestrations 905 serves as flow channels for the plurality of encapsulated leg members 906 and the central connection member 908 on the second, inward-facing side. The non-adherent drape 904 could also be used on the first side of the plurality of encapsulated leg members 906 and the central connection member 908.

Each of the encapsulated leg members 906 may be formed with a plurality of leg modules 910 with manipulation zones 912 between the plurality of leg modules 910. As with the manipulation zone 758 in FIGURES 7A-D, the manipulation zones 912 may facilitate movement of the plurality of encapsulated leg members 906 within the body cavity and may provide an easier location at which to cut the plurality of encapsulated leg members 906 when the open-cavity, reduced-pressure treatment device 902 is being sized for a particular application. In this regard, visual indicia 914 may be added on the non-adherent drape 904 to help the healthcare provider know where to cut the non-adherent drape 904 for different sizes of application within the cavity. The visual indicia 914 may comprise cut lines, or graduations, that preferably run through the manipulation zones 912. In some embodiments, the manipulation zones 912 may provide a convenient and easy location for cutting the open-cavity, reduced-pressure treatment device 902. In other embodiments, the leg members 906 may be cut anywhere along their length. For example, in some embodiments the leg members 906 may be cut for sizing through the leg modules 910, and the foam of the leg member manifold may then be pulled inward with respect to the film or drape to prevent exposure to the tissue site.

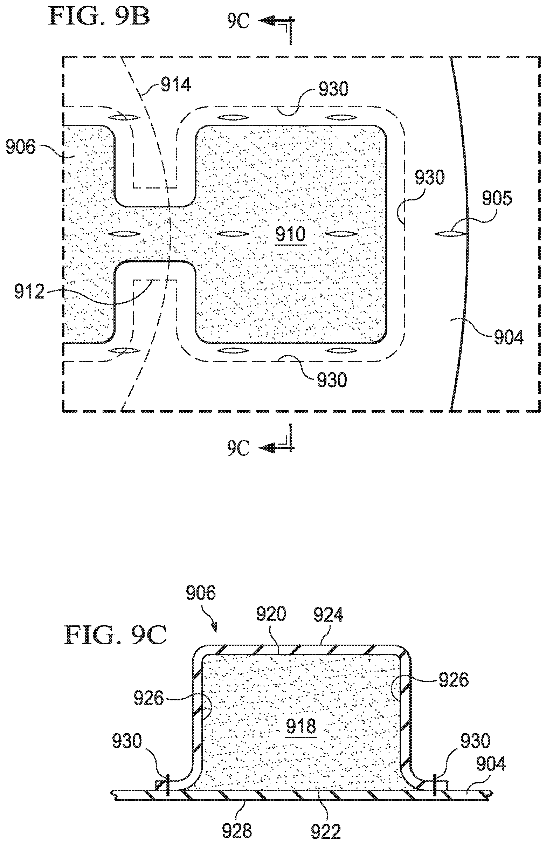

Referring to FIGURE 9C, a lateral cross section of a portion of an encapsulated leg member 906 is presented. The plurality of encapsulated leg members 906 are formed with a leg manifold member 918 having a first side 920 and a second, inward-facing (patient-facing) side 922. A first leg encapsulating member 924 covers the first side 920 of the leg manifold member 918 and covers a lateral zone or edge 926 of the leg manifold member 918. The second, inward-facing side 922 of the leg manifold member 918 is covered by a second leg encapsulating member 928, which in this embodiment is a portion of the non-adherent drape 904. The first leg encapsulating member 924 is coupled to the second leg encapsulating member 928 by any means known in the art, such as by welding (e.g., ultrasonic or RF), bonding, adhesives, cements, etc. In this illustrative embodiment, the first leg encapsulating member 924 and the second leg encapsulating member 928 are coupled by a weld 930. Referring to FIGURE 9B, the weld 930 is shown along the perimeter of the plurality of leg modules 910. In some embodiments, the weld 930 may be continuous along the perimeter of the leg module. In other embodiments the weld 930 may not be continuous. For example, the weld 930 may have small breaks along its length.

Referring again to FIGURE 9A, the central connection member 908 is formed analogously to the central connection member 712 in FIGURE 8. A first connection encapsulation member 934 and a second connection encapsulation member of the central connection member 908 are coupled along a peripheral edge 932 using a weld 933 or another coupling technique, such as those previously mentioned. The peripheral edge 932 is not sealed, however, proximate each of the encapsulated leg members 906 in order to provide a channel for fluid to flow from the plurality of encapsulated leg members 906 into the central connection member 908.

According to one illustrative approach to constructing the open-cavity, reduced-pressure treatment device 902, the non-adherent drape 904, which is already formed with the plurality of fenestrations 905 and that has visual indicia 914 is provided. The leg manifold member 918 is disposed adjacent the non-adherent drape 904. The central connection member 908 is disposed adjacent to the leg manifold member 918 or may be formed integral with leg manifold member 918. The first connection encapsulation member 934 is placed on the central connection member 908, and the first leg encapsulating member 924 is placed over the leg manifold member 918. The first connection encapsulation member 934 and the first leg encapsulating member 924 may be formed from an integral sheet. Next, the welds 930 and 933 are applied.

In an alternative embodiment for manufacturing an open-cavity, reduced-pressure treatment device, a first non-adherent drape 904, which includes fenestrations, may be provided and the leg manifold member 918 and the central connection member 908 disposed on the first non-adherent drape 904. A second non-adherent drape, which has fenestrations, is placed over the first non-adherent drape 904, the leg manifold member 918, and the central connection member 908. Next, a plurality of welds (e.g., thermal or RF or another coupling techniques used) are made, such as with the welds 930. The first non-adherent drape 904 and the second non-adherent drape may be cut to size before or after assembly. By using two drapes, the first non-adherent drape 904 and the second non-adherent drape may provide better distribution of reduced pressure and may ease the manufacturing process.

The fenestrations may be formed before or after assembly. The perimeter of the first non-adherent drape 904 and the second non-adherent drape may be welded. Other points may be welded between the drapes to form a single unit. In another alternative embodiment, the drapes may initially be placed and welded without fenestrations, and then fenestrations added to the drapes so that the fenestrations align. The fenestrations might also be formed using an electrical member that cuts and seals at the same time to form aligned, "button hole" fenestrations through the two drapes.

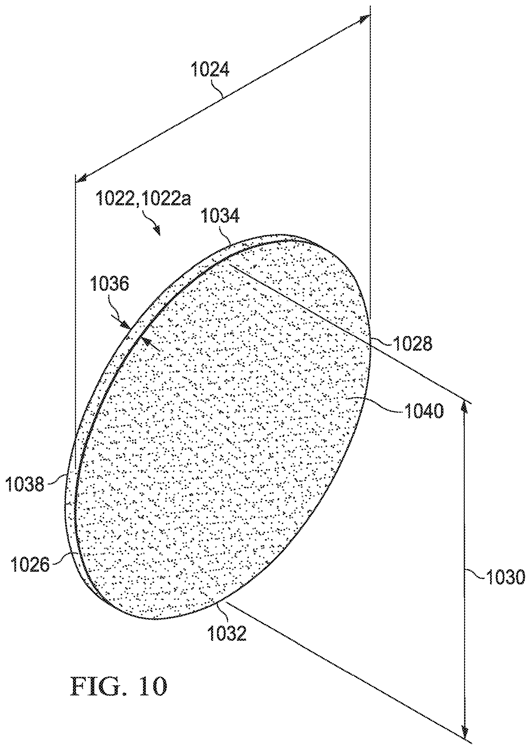

Referring to FIGURES 7A, 7D, and 10, in some example embodiments, the manifold pad 722 may be configured to distribute reduced pressure relative to the tissue 704 and other components of the system 100, such as, for example, the treatment device 702 or the treatment manifold 767. As such, the manifold pad 722 may be positioned proximate to and in fluid communication with the treatment device 702 or the treatment manifold 767. In some examples, the manifold pad 722 may be in fluid communication with the treatment manifold 767 through the non-adherent drape 748, or a portion of the non-adherent drape 748, such as the first non-adherent drape 786. Further, in some examples, the manifold pad 722 may be configured to be positioned in direct contact with the non-adherent drape 748 or the first non-adherent drape 786. Further, in some examples, the manifold pad 722 may be positioned proximate to and in fluid communication with the connection manifold member 754 or the central connection member 712. The sealing member 724 may be configured to cover the treatment manifold 767 and the manifold pad 722 at the tissue site 704.

Referring to FIGURES 7A and 10, in some example embodiments, the manifold pad 722 may include a lateral width 1024 extending from a first edge 1026 to an opposite second edge 1028 of the manifold pad 722. The lateral width 1024 may be configured to extend across the body-cavity opening 726 at the tissue site 704. Further, the manifold pad 722 may include a longitudinal length 1030 extending from a first end 1032 to an opposite second end 1034 of the manifold pad 722. In some embodiments, the longitudinal length 1030 may be greater than the lateral width 1024 and perpendicular to the lateral width 1024 of the manifold pad 722. The longitudinal length 1030 may be configured to be positioned along a closure line that may be formed by opposing sides of the body-cavity opening 726, such as, for example, an incision, wound edges, or a line of staples or sutures, etc. In some embodiments, the incision or body cavity opening may be formed by an open wound having wound edges. In some embodiments, the wound edges of the body-cavity opening 726 (which may be an abdominal cavity) may extend longitudinally. Further, the manifold pad 722 may include a thickness 1036 extending from a first side or surface 1038 to an opposite second side or surface 1040 of the manifold pad 722. The thickness 1036 of the manifold pad 722 may be perpendicular to both the lateral width 1024 and the longitudinal length 1030 of the manifold pad 722. In some embodiments, the term perpendicular may include orthogonal. In some embodiments, the thickness 1036 of the manifold pad 722 may range from about 5-25 mm, about 8-25 mm, about 8-16 mm, or about 5-16 mm. The first surface 1038 of the manifold pad 722 (which may be similar to the first surface 220 of the manifold layer 205) may be configured to face outward from or away from the tissue site 704 and, for example, the treatment device 702, the treatment manifold 767, or portions thereof. In some embodiments, the first surface 1038 may be configured to face towards the sealing member 724. Further, the second surface 1040 of the manifold pad 722 (which may be similar to the second surface 225 of the manifold layer 205) may be configured to face the tissue site 704 and, for example, the treatment device 702, the treatment manifold 767, or portions thereof. The first surface 1038 may mirror the second surface 1040 of the manifold pad 722, in some embodiments.

Referring to FIGURES 10-11, some embodiments of the manifold pad 722 may comprise or consist essentially of foam, such as open cell foam. Some manifold pad 722 embodiments may comprise the first surface 1038 and the second surface 1040, with the thickness 1036 of foam extending between the first surface 1038 and the second surface 1040. In some embodiments, the first surface 1038 may be configured to face outward, away from the tissue site (e.g. towards and/or contacting portions of the sealing member 724 over the manifold pad 722), and the second surface 1040 may be configured to face towards the tissue site (e.g. toward and/or contacting portions of the treatment device 702 underlying the manifold pad 722). In some embodiments, the foam of the manifold pad 722 may comprise a plurality of elongate, ovoidal or ellipsoidal pores (e.g. cells, not shown here, formed by the cell-structure of the foam), and in some embodiments such ovoidal pores may be formed by felting the foam. In some embodiments, the open-cell foam of the manifold pad 722 may have a cell-structure of open cells or pores. In some embodiments, (not shown) the manifold pad 722 may also include holes, which may provide additional porosity in addition to the porosity provided by the pores/cells of the foam of the manifold pad 722. In some embodiments, the manifold pad 722 may be configured for use in an abdominal wound, and may be configured to contract under application of negative pressure in such a way as to laterally close the abdominal wound, typically without significantly drawing down the thickness 1036 of the manifold pad 722.

In some embodiments, the foam of the manifold pad 722 may comprise felted foam, which may be formed by a felting process. As described herein, the terms "felting" or "felted" may refer to a porous material, such as the foam of the manifold pad 722, which has been treated or modified to impart a desired property to the porous material. Typically, the entire manifold pad 722 may be felted to the same firmness factor throughout, although in other embodiments the firmness factor may vary throughout the manifold pad 722. In some embodiments, the manifold pad 722 may be felted to a firmness factor greater than one and/or no more than about 5 (e.g. approximately 2-5, or approximately 3).

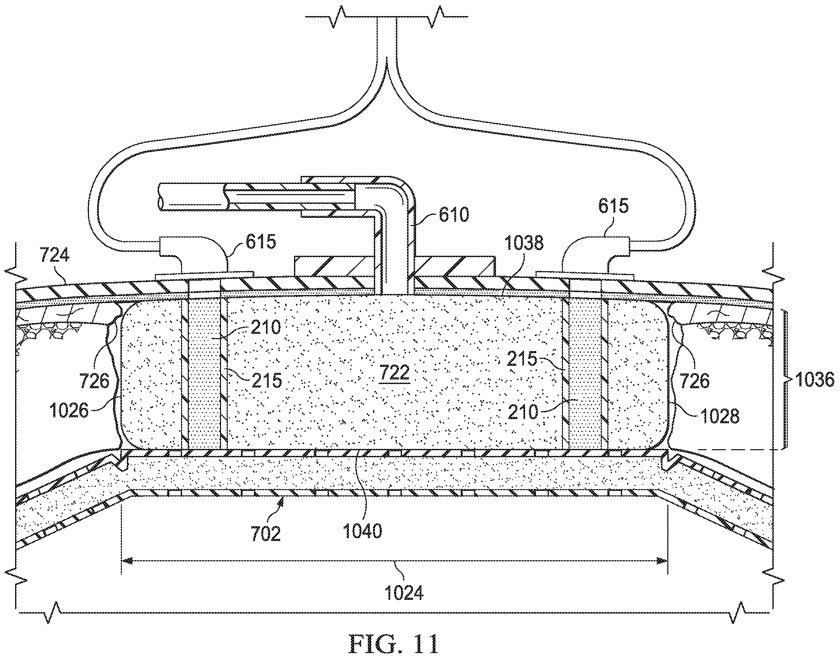

Some dressing embodiments, as shown in FIGURE 11 for example, may comprise a foam manifold pad 722 similar to the manifold layer 205 or manifold pad described herein. For example, the manifold pad 722 may comprise or consist essentially of the manifold layer 205. In some embodiments, the manifold pad 722 may be configured for use atop a treatment device 702, which may be similar to those examples described herein. In some embodiments, the manifold pad 722 may be configured for use with an abdominal wound, and may be configured to provide separate flowpaths or fluid pathways for instillation and negative-pressure therapy. For example, the manifold pad 722 may comprise one or more skinned channels 210 configured to deliver instillation fluid to the underlying treatment device without significant intrusion or seepage of such instillation fluid from the skinned channels 210 into the foam of the manifold pad 722. In some embodiments, the skin 215 of the skinned channels 210 may be configured to fluidly isolate the at least one skinned channel 210 from the open-cell foam as the channels 210 pass through the manifold pad 722. For example, the skinned walls of the channels 210 may be substantially liquid impermeable. In the embodiment shown in FIGURE 11, the skinned channels 210 comprise a plurality of vertical channels, which may be configured to extend from the first surface 1038 to the second surface 1040 of the manifold pad 722. Other embodiments of the manifold pad 722 (not shown here) may comprise one or more horizontal channels, for example extending from the first edge 1026 to the second edge 1028. Some embodiments may comprise at least one vertical channel and at least one horizontal channel, for example with the at least one vertical channel in fluid communication with the at least one horizontal channel and/or extending from the first surface 1038 to at least one horizontal channel.

In some embodiments, the configuration of the channels 210 and the interaction of the channels 210 with the instillation solution source may ensure that instillation only takes place through the skinned channels 210. The manifold pad 722 of FIGURE 11 may comprise open-cell foam configured to deliver negative-pressure therapy through an open porous structure to the underlying treatment device 702. In some embodiments, the open-cell foam may be felted, for example as described herein, and the skin 215 of the channels 210 may be integrally formed during such felting process. In some embodiments, the manifold pad 722 may be disposed between the cover or sealing member 724 and the treatment device 702, for example providing separate, independent fluid flowpaths for instillation (e.g. through the channels 210) and negative-pressure therapy (e.g. through the porous open-cell structure of the open-cell foam of the manifold pad 722) to the underlying treatment device 702. In some embodiments, the first fluid pathway or flowpath may further comprises the treatment device (e.g. the fluid pathway for instillation delivery from the instillation source to the tissue site may comprise the skinned channels 210 of the manifold pad 722 and the treatment device 702 in fluid communication with the channels 210), and the second fluid pathway or flowpath may further comprise the treatment device 702 (e.g. the fluid pathway for negative-pressure therapy from the negative-pressure source to the tissue site may comprise the porous structure of the open-cell foam of the manifold pad 722 and the treatment device 702 in fluid communication with the open-cell foam of the manifold pad 722). In some embodiments, the skinned channel portion of the first fluid pathway may be fluidly isolated from the open-cell foam portion of the second fluid pathway. For example, the skin 215 of the skinned channels 210 may isolate fluid flow through the channels 210 from fluid flow through the porous structure of the open-cell foam of the manifold pad 722, and vice versa.

In some embodiments, the dressing may further comprise the treatment device 702, which may be configured as a viscera protection layer to provide separation between the abdominal wall and the viscera and protect abdominal contents. In some embodiments, the treatment device 702 may have a film layer and may be configured to channel fluid radially under applied negative pressure. In some embodiments, the treatment device 702 may comprise a central hub and a plurality of elongate members extending outward from the central hub. In some embodiments, the plurality of elongate members may comprise 4-8 elongate members, which may be evenly spaced around the central fluid hub. In some embodiments, each elongate member may be in fluid communication with the central fluid hub. In some embodiments, the film layer may comprise a plurality of channels configured to allow radial fluid flow (e.g. flow from the elongate members to the central fluid hub). In some embodiments, the manifold pad 722 may be configured to be positioned proximate to and/or contacting the central fluid hub (e.g. between the central fluid hub of the treatment device 702 and the sealing member 724).

In some embodiments, the treatment device 702 may further comprise a treatment manifold, which may be attached to the film layer. In some embodiments, the treatment manifold may comprise open-cell foam. In some embodiments, the treatment manifold may be enveloped or encapsulated by the film layer, which may be a non-adherent drape with a plurality of fenestrations. In some embodiments, the treatment manifold may comprise a central connection manifold member and a plurality of leg manifold members extending outward from the central connection manifold member. In some embodiments, there may be 4-8 leg manifold members, which may be evenly spaced around the central connection manifold member. In some embodiments, each of the leg manifold members may be in fluid communication with the central connection manifold member.

Some dressing embodiments may further comprise a sealing member 724 configured to cover the manifold pad 722 and the treatment device 702 and to provide a pneumatic seal relative to the tissue site. In some embodiments, the sealing member 724 may be configured to be disposed over the treatment device 702 and manifold pad 722 to create a sealed space on the tissue site. In some embodiments, the manifold pad 722 may be configured so that the first surface 1038 faces towards and/or contacts the portions of the sealing member 724 disposed over the manifold pad 722, and the second surface 1040 faces towards and/or contacts the portions of the treatment device 702 underlying the manifold pad 722. In some embodiments, the manifold pad 722 may be configured to distribute applied negative pressure to the treatment device 702 (and thereby to the tissue site), for example through the porous structure of the foam. In some embodiments, the manifold pad 722 may be configured to distribute instillation fluid to the treatment device 702 (and thereby to the tissue site), for example through one or more skinned channels 210. In some embodiments, the fluid distribution pathway for instillation fluid solution through the manifold pad 722 may be entirely separate from the fluid distribution pathway for negative-pressure through the manifold pad 722. In some embodiments, the treatment device 702 may comprise a single film layer, and the single film layer may be attached to a surface of the treatment manifold. In some embodiments, the film layer may comprise a plurality of fenestrations. In some embodiments, the treatment device 702 may be similar to those described in more detail with respect to FIGURES 7A-10.

System embodiments may comprise a dressing similar to those described herein, a negative-pressure source fluidly coupled to the dressing (e.g. to the open-cell foam of the manifold pad 722), and an instillation solution source fluidly coupled to the dressing (e.g. to the skinned channels 210 of the manifold pad 722). In some embodiments, the negative-pressure source may fluidly couple to the sealed space formed by the dressing by contacting the open-cell foam of the manifold pad on the first surface. For example, the negative-pressure source may fluidly communicate with the open-cell foam of the manifold pad 722 through the sealing member 724 (e.g. via negative-pressure interface 610), and the applied negative pressure may be distributed through the foam structure of the manifold pad 722, through the treatment device 702, to the tissue site. In some embodiments, the dressing may be used on a tissue site with an incision, and the application of negative pressure to the manifold pad 722 may draw the incision or wound edges closer together to promote closure of the wound tissue site. In some embodiments, instillation solution source may fluidly communicate with the skinned channels 210 through the sealing member 724 (e.g. via one or more instillation interface 615), with instillation fluid distributed through the skinned channels 210 of the manifold pad 722 to the treatment device 702, and thereby to the tissue site. In some embodiments, the sealing member 724 may include apertures or openings that are configured to align with the one or more of the skinned channels 210 (e.g. vertical channels 305), allowing introduction of instillation fluid from the instillation solution source, through the sealing member, to the channels 210 without significant flow into the foam of the manifold pad 722.

For treating a tissue site, for example using a manifold pad 722, treatment device 702, dressing and/or system similar to those described herein, exemplary methods may comprise: applying a treatment device 702, similar to those described herein, to the tissue site; applying a manifold pad 722, similar to those described herein, over the treatment device 702; and applying a sealing member 724 over the manifold pad 722 to form a sealed space on the tissue site. In some embodiments, the manifold pad 722 may be applied with the first surface 1038 substantially parallel to a portion of the tissue site underlying the manifold pad 722.

Another illustrative embodiment for using an open-cavity, reduced-pressure treatment device or system according to this disclosure will now be presented. The system is particularly suitable for temporary bridging of abdominal wall openings where primary closure may not be readily possible and or repeat abdominal entries are necessary. The illustrative system described here may be used with open abdominal wounds, with exposed viscera, including but not limited to abdominal compartment syndrome. Before applying the system, typically hemostasis should be achieved.

In deploying the open-cavity, reduced-pressure treatment system, the reduced-pressure treatment device preferably covers all exposed viscera and preferably separates completely the viscera from contact with the abdominal wall. For example, the lower surface of the reduced pressure treatment device, such as drape 904, can be sized and shaped to permit coverage. The reduced-pressure treatment device may be placed over the omentum or exposed internal organs, and carefully tucked between the abdominal wall and internal organs. In doing so, the healthcare provider may use the reduced-pressure treatment device to completely separate the abdominal wall from the internal organs.

To prepare for deployment of the system, any sharp edges or bone fragments are eliminated from wound area or covered. The abdominal wound is irrigated and the periwound area cleaned. The periwound tissue at the epidermis is typically dried before further application.

The reduced-pressure treatment device is then sized by determining the appropriate size and cutting. The reduced-pressure treatment device is initially unfolded in a sterile field. Either side of the reduced-pressure treatment device may be placed on the omentum or viscera. The reduced-pressure treatment device is gently placed over the open abdominal cavity. The orientation of the reduced-pressure treatment device for the specific application is determined. If the reduced-pressure treatment device will be placed around tubes, drains or the falciform ligament, the reduced-pressure device is cut only between the plurality of encapsulated leg members. The reduced-pressure treatment device is placed in the proper orientation before cutting.

The reduced-pressure treatment device is then folded to size and used that way or may be cut. The healthcare provider holds the reduced-pressure treatment device by the edge and slightly lifts the reduced-pressure treatment device. The reduced-pressure treatment device is slowly lowered into the paracolic gutter with one hand and the other hand is used to gently and evenly work the reduced-pressure treatment device down. The healthcare provider folds any excess portions of the reduced-pressure treatment device up and over onto itself. The healthcare provider continues to place the reduced-pressure treatment device between abdominal wall and internal organs throughout the abdominal compartment. The healthcare provider preferably provides full coverage of all viscera. The reduced-pressure treatment device may then be cut as needed for sizing outside of the wound.

To size the device, the reduced-pressure treatment device can be cut through center of one of the large manifold squares, or leg modules, using sterile scissors. In this illustrative embodiment, the cut is not made through the manipulation zone, but through the leg module. The healthcare provider then pinches the remaining half of the foam square, or leg module, and the adjacent, inboard manipulation zone through the encapsulating member with one hand and pulls the manifold material. The manifold material in the leg module and the manipulation zone will separate at the next square, or leg module. This will ensure that edges of the reduced-pressure treatment device cover the otherwise exposed manifold edge. The manifold material, e.g., foam, preferably does not contact organs.

The manifold pad that is to be placed on top of the central connection member is next prepared. In this embodiment, the manifold pad may be a perforated foam manifold that has perforations to help tear the manifold to the desired size. The manifold pad preferably fits directly over the reduced-pressure treatment device while still being in contact with wound edges. The manifold pad generally should not contact intact skin. Two or more manifolds may be used (e.g. stacked) in some instances. Then, the sized manifold pad is gently placed into the wound cavity over the reduced-pressure treatment device. The healthcare provider preferably takes care to avoid the manifold pad going below the level of the abdominal incision or wound.

A drape, or over-drape or sealing member, is then applied. To apply the drape, a backing is removed from an adhesive layer on one side of the drape and the drape is applied. The drape covers the manifold and a portion of intact epidermis. Preferably the drape covers at least an 8-10 centimeter border of intact periwound tissue. Additional drape material may be added to seal any difficult areas.

The reduced-pressure interface, or negative-pressure interface pad, is then added. The healthcare provider chooses an application site. The site is chosen to optimize fluid flow as well as to facilitate easy tubing positioning. The healthcare provider pinches the drape and cuts a 2.5 cm hole (preferably not a slit) through the drape. The negative-pressure interface pad, which has a central disc and a surrounding outer adhesive skirt, is applied. The negative-pressure interface pad is applied by removing backing layers on an inward-facing surface of the interface pad to expose an adhesive. The negative-pressure interface pad opening in the central disc is placed directly over the hole in the drape. Pressure is gently applied on the central disc and the outer skirt to ensure complete adhesion of the interface pad. One or more stabilization layers may then be removed from a first side of the skirt. In some embodiments, one or more instillation interface 615 may similarly be applied to be in fluid communication with one or more skinned channels 210 in the manifold pad (e.g. adhered over one or more holes in the drape cover corresponding to one or more vertical skinned channels). The system is now ready for the application of reduced pressure and/or instillation, which may take place using entirely separate fluid pathways.

The systems, apparatuses, and methods described herein may provide significant advantages. For example, disclosed manifold pads or dressings may distribute negative pressure to a tissue site, which may facilitate removal of fluids, such as exudate, from the tissue site. Some embodiments of the manifold pad or dressing may be configured to provide instillation to the tissue site. Having a configuration that allows for separate fluid pathways for instillation and negative-pressure therapy may allow for instillation solution to be directed precisely to the treatment device and/or tissue site, for example without some portion of the instillation solution being retained in the foam of the manifold pad. This approach may speed instillation, by reducing or preventing waste instillation solution being retained in the open-cell foam and allowing instillation solution to by-pass the open-cell foam. Additionally, this approach may prevent or reduce the likelihood of contamination of the instillation solution, for example by exudate remaining in the foam after negative-pressure therapy. These and other advantages may arise from the disclosure.

If something is described as "exemplary" or an "example", it should be understood that refers to a non-exclusive example. The terms "about" or "approximately" or the like, when used with a number, may mean that specific number, or alternatively, a range in proximity to the specific number as understood by persons of skill in the art field (for example, +/-10%). Use of broader terms such as "comprises", "includes", and "having" should be understood to provide support for narrower terms such as "consisting of", "consisting essentially of", and "comprised substantially of". Use of the term "optionally", "may", "might", "possibly", "could", "can", "would", "should", "preferably", "typically", "often" and the like with respect to any element, component, feature, characteristic, etc. of an embodiment means that the element, component, feature, characteristic, etc. is not required, or alternatively, the element, component, feature, characteristic, etc. is required, both alternatives being within the scope of the embodiment(s). Such element, component, feature, characteristic, etc. may be optionally included in some embodiments, or it may be excluded (e.g. forming alternative embodiments, all of which are included within the scope of disclosure). Section headings used herein are provided for consistency and convenience, and shall not limit or characterize any invention(s) set out in any claims that may issue from this disclosure. If a reference numeral is used to reference a specific example of a more general term, then that reference numeral may also be used to refer to the general term (or vice versa).

While shown in a few illustrative embodiments, a person having ordinary skill in the art will recognize that the , , methods described herein are susceptible to various changes and modifications that fall within the scope of the appended claims. Moreover, descriptions of various alternatives using terms such as "or" do not require mutual exclusivity unless clearly required by the context, and the indefinite articles "a" or "an" do not limit the subject to a single instance unless clearly required by the context. Components may be also be combined or eliminated in various configurations for purposes of sale, manufacture, assembly, or use. For example, in some configurations the treatment device, manifold pad, and/or sealing member may be eliminated or separated from other components for manufacture or sale. In other example configurations, the controller may also be manufactured, configured, assembled, or sold independently of other components.

## Claims

1. A method of forming a manifold pad, comprising:
providing open-cell foam (405);
disposing one or more heating cores (410) within the foam;
heating the heating cores (410); and
felting the open-cell foam (405);
wherein felting the open-cell foam (405) while heating the heating cores (410) forms skinned channels (210) having a skin that is substantially liquid impermeable and integrally formed with the felted open-cell foam (405);
wherein providing open-cell foam (405) comprises providing two or more open-cell foam blanks (405); disposing one or more heating cores (410) within the foam comprises disposing the one or more heating cores (410) between the open-cell foam blanks (405); and felting the open-cell foam (405) joins the two or more open-cell foam blanks (405) into a unitary whole.

2. A method of forming a manifold pad, comprising:
providing open-cell foam (405);
disposing one or more heating cores (410) within the foam;
heating the heating cores (410); and
felting the open-cell foam (405);
wherein felting the open-cell foam (405) while heating the heating cores (410) forms skinned channels (210) having a skin that is substantially liquid impermeable and integrally formed with the felted open-cell foam (405);
wherein providing open-cell foam (405) comprises providing a foam blank; and disposing the heating cores with the foam comprises forming holes in the foam blank and inserting the heating cores into the holes.

3. The method of claim 1 or claim 2, wherein the open-cell foam (405) is felted to a firmness factor of 2-5.

4. The method of claim 1, further comprising removing the heating cores (410) from the felted foam (405).

5. The method of claim 1, wherein felting the foam (405) comprises compressing the foam approximately perpendicular to a longitudinal axis of the heating cores (410).

6. The method of claim 1, wherein the foam is felted to a firmness factor greater than 1 and less than 5.

7. The method of claim 1, further comprising cutting a core channel to fluidly couple a first surface of the foam to at least one skinned channel (210).

8. The method of claim 7, wherein cutting a core channel comprises using a heated cutting element to form the core channel.

9. The method of claim 1, further comprising disposing a vertical heating core within one or more of the foam blanks (405), wherein the vertical heating core extends from the first surface to one or more of horizontal heating cores (410).

10. The method of claim 1, wherein felting the open-cell foam (405) further comprises providing one or more felting platens (415) configured to felt the foam blanks (405).

11. The method of claim 10, wherein the one or more heating cores (410) are integral to the platens (415), and disposing the heating cores within the open-cell foam (405) occurs during felting of the open-cell foam (405).

12. The method of claim 1, wherein felting the open-cell foam (405) comprises disposing the open-cell foam (405) between platens (415); heating the platens (415); and compressing the open-cell foam (405) between the platens (415).

## Patentansprüche

1. Ein Verfahren zum Ausbilden eines Verteilerkissens, aufweisend:
Bereitstellen eines offenzelligem Schaumstoffs (405);
Anordnen eines oder mehrerer Heizkerne (410) innerhalb des Schaumstoffs;
Erhitzen der Heizkerne (410); und
Filzen des offenzelligen Schaumstoffs (405);
wobei das Filzen des offenzelligen Schaumstoffs (405) unter Erhitzen der Heizkerne (410) mit einer Haut versehene Kanäle (210), die eine Haut aufweisen, die im Wesentlichen flüssigkeitsundurchlässig ist und mit dem filzten offenzelligen Schaumstoff (405) einstückig ausgebildet ist;
wobei das Bereitstellen von offenzelligem Schaumstoff (405) das Bereitstellen von zwei oder mehr Rohlingen (405) des offenzelligen Schaumstoff aufweist; das Anordnen eines oder mehrerer Heizkerne (410) innerhalb des Schaumstoffs das Anordnen des einen oder der mehreren Heizkerne (410) zwischen den Rohlingen (405) des offenzelligen Schaumstoffs aufweist; und das Filzen des offenzelligen Schaumstoffs (405) die zwei oder mehr Rohlinge (405) des offenzelligen Schaumstoffs zu einem einheitlichen Ganzen verbindet.

2. Ein Verfahren zum Ausbilden eines Verteilerkissens, aufweisend:
Bereitstellen eines offenzelligem Schaumstoffs (405);
Anordnen eines oder mehrerer Heizkerne (410) innerhalb des Schaumstoffs;
Erhitzen der Heizkerne (410); und
Filzen des offenzelligen Schaumstoffs (405);
wobei das Filzen des offenzelligen Schaumstoffs (405) unter Erhitzen der Heizkerne (410) mit einer Haut versehene Kanäle (210), die eine Haut aufweisen, die im Wesentlichen flüssigkeitsundurchlässig ist und mit dem filzten offenzelligen Schaumstoff (405) einstückig ausgebildet ist;
wobei das Bereitstellen des offenzelligen Schaumstoffs (405) das Bereitstellen eines Schaumstoffrohlings aufweist; und das Anordnen der Heizkerne mit dem Schaumstoff das Ausbilden von Löchern in dem Schaumstoffrohling und ein Einsetzen der Heizkerne in die Löcher aufweist.

3. Das Verfahren nach Anspruch 1 oder 2, wobei der offenzellige Schaumstoff (405) auf einen Härtefaktor von 2-5 gefilzt wird.

4. Das Verfahren nach Anspruch 1, ferner aufweisend ein Entfernen der Heizkerne (410) aus dem gefilzten Schaumstoff (405).

5. Das Verfahren nach Anspruch 1, wobei das Filzen des Schaumstoffs (405) ein Komprimieren des Schaumstoffs etwa senkrecht zu einer Längsachse der Heizkerne (410) aufweist.

6. Das Verfahren nach Anspruch 1, wobei der Schaumstoff auf einen Härtefaktor größer als 1 und kleiner als 5 gefilzt wird.

7. Das Verfahren nach Anspruch 1, ferner aufweisend ein Schneiden eines Kernkanals, um eine erste Oberfläche des Schaumstoffs mit mindestens einem mit Haut versehenen Kanal (210) fluidisch zu koppeln.

8. Das Verfahren nach Anspruch 7, wobei das Schneiden eines Kernkanals ein Verwenden eines erhitzten Schneidelements aufweist, um den Kernkanal auszubilden.

9. Das Verfahren nach Anspruch 1, ferner aufweisend das Anordnen eines vertikalen Heizkerns innerhalb eines oder mehreren der Schaumstoffrohlinge (405), wobei sich der vertikale Heizkern von der ersten Oberfläche zu einem oder mehreren der horizontalen Heizkerne (410) erstreckt.

10. Das Verfahren nach Anspruch 1, wobei das Filzen des offenzelligen Schaumstoffs (405) ferner das Bereitstellen einer oder mehrerer Filzaufspannplatten (415) aufweist, die konfiguriert sind, um die Schaumstoffrohlinge (405) zu filzen.

11. Verfahren nach Anspruch 10, wobei der eine oder die mehreren Heizkerne (410) mit den Aufspannplatten (415) integral sind und das Anordnen der Heizkerne in dem offenzelligen Schaumstoff (405) während des Filzens des offenzelligen Schaumstoffs (405) erfolgt.

12. Das Verfahren nach Anspruch 1, wobei das Filzen des offenzelligen Schaumstoffs (405) das Anordnen des offenzelligen Schaumstoffs (405) zwischen Aufspannplatten (415) aufweist; Erhitzen der Aufspannplatten (415); und Komprimieren des offenzelligen Schaumstoffs (405) zwischen den Aufspannplatten (415).

## Revendications

1. Procédé de formation d'un tampon de collecteur, comprenant :
la fourniture de mousse à alvéoles ouvertes (405) ;
la mise en place d'un ou plusieurs noyaux de chauffage (410) au sein de la mousse ;
le chauffage des noyaux de chauffage (410) ; et
le feutrage de la mousse à alvéoles ouvertes (405) ;
dans lequel le feutrage de la mousse à alvéoles ouvertes (405) tout en chauffant les noyaux de chauffage (410) forme des canaux à peau (210) ayant une peau qui est sensiblement imperméable aux liquides et formée d'un seul tenant avec la mousse à alvéoles ouvertes (405) feutrée ;
dans lequel la fourniture de mousse à alvéoles ouvertes (405) comprend la fourniture de deux ébauches de mousse à alvéoles ouvertes (405) ou plus ; la mise en place d'un ou plusieurs noyaux de chauffage (410) au sein de la mousse comprend la mise en place du ou des noyaux de chauffage (410) entre les ébauches de mousse à alvéoles ouvertes (405) ; et le feutrage de la mousse à alvéoles ouvertes (405) joint les deux ébauches de mousse à alvéoles ouvertes (405) ou plus en un ensemble solidaire.

2. Procédé de formation d'un tampon de collecteur, comprenant :
la fourniture de mousse à alvéoles ouvertes (405) ;
la mise en place d'un ou plusieurs noyaux de chauffage (410) au sein de la mousse ;
le chauffage des noyaux de chauffage (410) ; et
le feutrage de la mousse à alvéoles ouvertes (405) ;
dans lequel le feutrage de la mousse à alvéoles ouvertes (405) tout en chauffant les noyaux de chauffage (410) forme des canaux à peau (210) ayant une peau qui est sensiblement imperméable aux liquides et formée d'un seul tenant avec la mousse à alvéoles ouvertes (405) feutrée ;
dans lequel la fourniture de mousse à alvéoles ouvertes (405) comprend la fourniture d'une ébauche de mousse ; et la mise en place des noyaux de chauffage avec la mousse comprend la formation de trous dans l'ébauche de mousse et l'insertion des noyaux de chauffage dans les trous.

3. Procédé selon la revendication 1 ou la revendication 2, dans lequel la mousse à alvéoles ouvertes (405) est feutrée à un facteur de fermeté de 2 à 5.

4. Procédé selon la revendication 1, comprenant en outre le retrait des noyaux de chauffage (410) de la mousse feutrée (405).

5. Procédé selon la revendication 1, dans lequel le feutrage de la mousse (405) comprend la compression de la mousse à peu près perpendiculaire à un axe longitudinal des noyaux de chauffage (410).

6. Procédé selon la revendication 1, dans lequel la mousse est feutrée à un facteur de fermeté supérieur à 1 et inférieur à 5.

7. Procédé selon la revendication 1, comprenant en outre la découpe d'un canal de noyau pour accoupler fluidiquement une première surface de la mousse à au moins un canal à peau (210).

8. Procédé selon la revendication 7, dans lequel la découpe d'un canal de noyau comprend l'utilisation d'un élément de coupe chauffé pour former le canal de noyau.

9. Procédé selon la revendication 1, comprenant en outre la mise en place d'un noyau de chauffage vertical au sein d'une ou plusieurs des ébauches de mousse (405), dans lequel le noyau de chauffage vertical s'étend de la première surface jusqu'à un ou plusieurs des noyaux de chauffage horizontaux (410).

10. Procédé selon la revendication 1, dans lequel le feutrage de la mousse à alvéoles ouvertes (405) comprend en outre la fourniture d'un ou plusieurs plateaux de feutrage (415) conçus pour feutrer les ébauches de mousse (405).

11. Procédé selon la revendication 10, dans lequel le ou les noyaux de chauffage (410) sont solidaires des plateaux (415), et la mise en place des noyaux de chauffage au sein de la mousse à alvéoles ouvertes (405) se produit pendant le feutrage de la mousse à alvéoles ouvertes (405).

12. Procédé selon la revendication 1, dans lequel le feutrage de la mousse à alvéoles ouvertes (405) comprend la mise en place de la mousse à alvéoles ouvertes (405) entre les plateaux (415) ; le chauffage des plateaux (415) ; et la compression de la mousse à alvéoles ouvertes (405) entre les plateaux (415).
